(19) Europäisches Patentamt
European Patent Office
Office européen des brevets

(11) EP 0 700 905 B1

(12) EUROPÄISCHE PATENTSCHRIFT

(45) Veröffentlichungstag und Bekanntmachung des Hinweises auf die Patenterteilung:
**31.10.2001 Patentblatt 2001/44**

(51) Int Cl.[7]: **C07D 231/54**, C07D 231/56, A61K 31/415

(21) Anmeldenummer: **95112270.4**

(22) Anmeldetag: **04.08.1995**

(54) **Tricyclische Pyrazol-Derivate**

Tricyclic pyrazole derivatives

Dérivés tricycliques de pyrazole

(84) Benannte Vertragsstaaten:
**AT BE CH DE DK ES FR GB GR IE IT LI LU MC NL PT SE**

(30) Priorität: **12.08.1994 CH 249094**

(43) Veröffentlichungstag der Anmeldung:
**13.03.1996 Patentblatt 1996/11**

(73) Patentinhaber: **F. HOFFMANN-LA ROCHE AG
4002 Basel (CH)**

(72) Erfinder: **Wichmann, Jürgen
D-79585 Steinen (DE)**

(74) Vertreter: **Poppe, Regina et al
F.Hoffmann-La Roche AG
Patent Department(PLP),
124 Grenzacherstrasse
4070 Basel (CH)**

(56) Entgegenhaltungen:
**DE-A- 1 770 923** **DE-A- 2 249 644**

- **JOURNAL OF MEDICINAL CHEMISTRY, Bd. 20, Nr. 5, 1977 Seiten 664-669, K. RAMALINGAM ET AL. 'Synthesis and Antimicrobial Activity ...'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 700 905 B1

## Beschreibung

Die vorliegende Erfindung betrifft tricyclische Pyrazol-Derivate, im speziellen betrifft sie tricyclische 1-Aminoethylpyrazol-Derivate der allgemeinen Formel

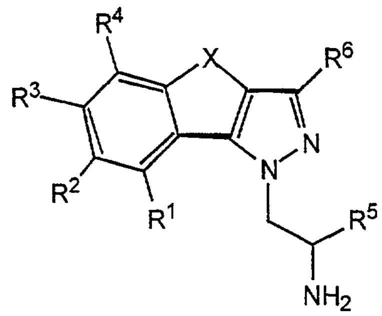

I

worin

| | |
|---|---|
| $R^1$ bis $R^4$ | je Wasserstoff, Hydroxy, Halogen, niederes Alkyl, niederes Alkoxy oder Phenyl; |
| $R^5$ | niederes Alkyl; |
| $R^6$ | Wasserstoff, niederes Alkyl oder niederes Alkoxy; |
| X | $-(CR^7R^8)_n-$ oder -CH=CH-; |
| $R^7$ und $R^8$ | Wasserstoff oder niederes Alkyl und |
| n | 1 oder 2 bedeuten, |

sowie pharmazeutisch annehmbare Salze von basischen Verbindungen der allgemeinen Formel I.

Diese Verbindungen und Salze sind neu und zeichnen sich durch wertvolle pharmakologische Eigenschaften aus.

Gegenstand der vorliegenden Erfindung sind Verbindungen der allgemeinen Formel I und pharmazeutisch annehmbare Salze davon als solche und als pharmazeutische Wirkstoffe, die Herstellung der Verbindungen der allgemeinen Formel I und ihrer Salze, ferner Arzneimittel, die diese Verbindungen und Salze enthalten und die Herstellung dieser Arzneimittel, sowie die Verwendung von Verbindungen der allgemeinen Formel I und von pharmazeutisch anwendbaren Salzen davon bei der Bekämpfung bzw. Verhütung von Krankheiten bzw. bei der Verbesserung der Gesundheit, insbesondere bei der Bekämpfung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc., Schädigungen des Nervensystems durch Trauma, Schlaganfall, neurodegenerative Erkrankungen etc., cardiovasculären Störungen, wie Bluthochdruck, Thrombose, Schlaganfall etc. und gastrointestinalen Störungen, wie Dysfunktion der Motilität des Gastrointestinaltrakts bzw. zur Herstellung entsprechender Arzneimittel.

Weiterhin sind Gegenstand der Erfindung die Verbindungen der allgemeinen Formel

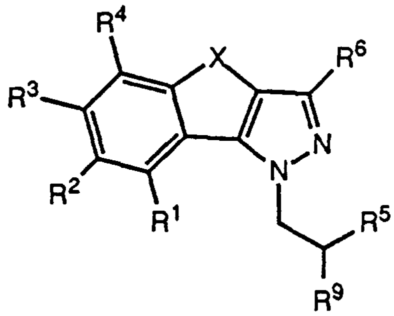

II

worin $R^1$ bis $R^6$ die oben genannten Bedeutungen haben und $R^9$ eine Azidgruppe, eine Hydroxygruppe oder eine geschützte Aminogruppe bedeutet.

In der Offenlegungsschrift DE 1 770 923 werden tetracyclische Pyrazolverbindungen beschrieben, die Psychotherapeutika sind und als Sedativa, Relaxantien, antikonvulsive Mittel, Mittel gegen die Parkinson-Krankheit, Hypnotika, Analgetika, hustenstillende Mittel, entzündungshemmende Mittel, Inhibitoren des autonomen Nervensystems und Antispastika verwendet werden können.

Die Offenlegungsschrift DE 2 249 644 beschreibt tricyclische Pyrazolverbindungen mit einem nieder-Alkyl-Substituenten am N-Atom des Pyrazolringes. Diese Verbindungen haben hypotensive/antihypertensive Wirkungen.

Die Verbindungen der Formel II sind wichtige Zwischenprodukte zur Herstellung der pharmazeutisch wertvollen Verbindungen der allgemeinen Formel I.

Falls in Formel I keines der Symbole $R^1$ bis $R^6$ ein Asymmetriezentrum aufweist, können die erfindungsgemässen Verbindungen als Enantiomere vorliegen, andernfalls sind verschiedene Diastereomere möglich. Die Erfindung umfasst sämtliche möglichen Stereoisomeren sowie auch Gemische davon.

Der in der vorliegenden Beschreibung verwendete Ausdruck "nieder" bezeichnet Reste mit höchstens 7, vorzugsweise bis 4 Kohlenwasserstoffatomen, "Alkyl" bezeichnet geradkettige, verzweigte oder cyclische gesättigte Kohlenwasserstoffreste wie Methyl, Ethyl, Propyl, Isopropyl oder t-Butyl, und "Alkoxy" bezeichnet eine über ein Sauerstoffatom gebundene Alkylgruppe, "Halogen" kann Cl, Br, F oder J bedeuten.

Der Ausdruck "pharmazeutisch annehmbare Salze" umfasst Salze mit anorganischen und organischen Säuren, wie Salzsäure, Bromwasserstoffsäure, Salpetersäure, Schwefelsäure, Phosphorsäure, Zitronensäure, Ameisensäure, Fumarsäure, Maleinsäure, Essigsäure, Bernsteinsäure, Weinsäure, Methansulfonsäure, p-Toluolsulfonsäure und dergleichen.

$R^5$ bedeutet nieder-Alkyl, vorzugsweise Methyl.

Besonders bevorzugt sind in diesem Fall Verbindungen, worin $R^2$ Methyl oder Methoxy, X -CH2- oder -C$(CH_3)_2$- und $R^1$, $R^3$, $R^4$ und $R^6$ Wasserstoff bedeutet.

Einige im Rahmen der vorliegenden Erfindung ganz besonders bevorzugte Vertreter der durch die allgemeine Formel I definierten Stoffklasse sind:

(RS)-2-(7-Methoxy-1,4-dihydro-indeno[2,1-c]pyrazol-1-yl)-1-methylethylamin fumarat (1:1),

(S)-2-(7-Methoxy-1,4-dihydro-indeno[2,1-c]pyrazol-1-yl)-1-methylethylamin fumarat (1:1),

(S)-2-(4,4,7-Trimethyl-1,4-dihydro-indeno[2,1-c]pyrazol-1-yl)-1-methylethylamin fumarat (1:1),

(S)-2-(7-Methoxy-4,4-dimethyl-1,4-dihydro-indeno[2,1-c]pyrazol-1-yl)-1-methyl-ethylamin fumarat (1:1),

(RS)-2-(7-Methoxy-4,4-dimethyl-1,4-dihydro-indenol[2,1-c]pyrazol-1-yl)-1-methyl-ethylamin fumarat (1:1),

(RS)-2-(7-Ethoxy-1,4-dihydro-indeno[2,1-c]pyrazol-1-yl)-1-methylethylamin fumarat (1:1);

(R)-2-(7-Methoxy-1,4-dihydro-indeno[2,1-c]pyrazol-1-yl)-1-methylethylamin fumarat (1:1).

(RS)-2-(8-Methoxy-1-H-benz[g]indazol-1-yl)-1-methyl-ethylamin fumarat (1:0,5)

Die Verbindungen der allgemeinen Formel I sowie ihre pharmazeutisch annehmbaren Salze können erfindungsgemäss hergestellt werden, indem man

a) eine Verbindung der allgemeinen Formel

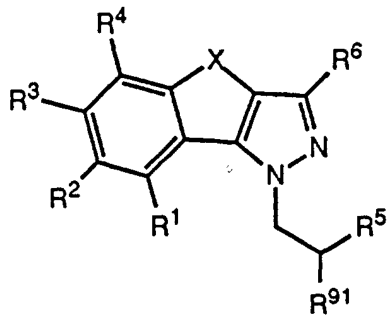

IIa

worin $R^1$ bis $R^6$ die oben angegebene Bedeutung haben und $R^{91}$ einen in eine Aminogruppe überführbaren Rest bedeutet, in eine entsprechende Aminoverbindung überführt und

b) erwünschtenfalls die erhaltene Verbindung der Formel I in ein pharmazeutisch annehmbares Salz überführt.

Die Verbindungen der allgemeinen Formel IIa, worin $R^{91}$ einen in eine Aminogruppe überführbaren Rest, vorzugsweise eine Azidgruppe, eine Acetylaminogruppe oder eine andere geschützte Aminogruppe bedeutet, können wie weiter unten beschrieben, nach an sich bekannten Methoden hergestellt werden.

Ist der Rest $R^{91}$ eine Azidgruppe, erfolgt die Herstellung der Verbindungen der Formel I durch Reduktion. Diese kann in an sich bekannter Weise mit komplexen Hydriden, wie z.B. Lithiumaluminiumhydrid oder durch katalytische Hydrierung an Metallkatalysatoren, wie z.B. Platin oder Palladium, durchgeführt werden. Verwendet man Lithiumaluminiumhydrid als Reduktionsmittel, sind vor allem wasserfreier Aether oder Tetrahydrofuran als Lösungsmittel geeignet.

Die katalytische Hydrierung an Metallkatalysatoren, z.B. Platin oder Palladium, erfolgt zweckmässigerweise bei Raumtemperatur. Als Lösungsmittel sind dazu besonders geeignet: Wasser, Alkohole, Essigester, Dioxan oder Gemische dieser Lösungsmittel. Die Hydrierung erfolgt unter Wasserstoffatmosphäre zweckmässigerweise in einem Autoklaven oder in einer Schüttelapparatur.

Bedeutet $R^{91}$ eine Acetylaminogruppe oder eine andere geschützte Aminogruppe, wie z.B.Trifluoracetylamino, erfolgt eine Ueberführung in die entsprechende Aminoverbindung durch Hydrolyse.

Die Hydrolyse zu den entsprechenden Aminoverbindungen der allgemeinen Formel I erfolgt nach an sich bekannten Methoden. Es eignen sich dafür Metallhydroxide, beispielsweise Natrium- oder Kaliumhydroxid, die in Anwesenheit von Wasser und einem mit Wasser mischbaren organischen Lösungsmittel, wie einem Alkohol, Ethylenglykol oder dergleichen, zu den Verbindungen der Formel I hydrolysieren.

Die Ueberführung der Verbindungen der Formel I in ihre Säureadditionssalze erfolgt im letzten Arbeitsgang, d.h. nach der Hydrierung oder Hydrolyse der Verbindungen der Formel I.

Aus Stabilitätsgründen eignen sich die Salze der Fumarsäure besonders gut für eine pharmazeutische Anwendung. Aber auch alle anderen in der Beschreibung erwähnten Säuren bilden pharmakologisch annehmbare Salze. Die Salzbildung erfolgt nach jedem Fachmann geläufigen Methoden bei Raumtemperatur, als Lösungsmittel sind Alkohol-Ethergemische besonders geeignet.

Die Herstellung der Zwischenprodukte der Formel II, die für die Synthese der Verbindungen der allgemeinen Formel I benötigt werden, ist im Schema 1 und 2 dargestellt.

Hierbei haben alle Substituenten $R^1$ bis $R^5$ die in der Formel I angegebenen Bedeutungen, $R^{61}$ bedeutet Wasserstoff oder niederes Alkyl und Me bedeutet Methyl. $X^1$ hat ebenfalls die in der Formel I für X angegebene Bedeutung, ausser für Verbindungen mit X = -CH=CH-, deren Herstellung das Schema 3 zeigt.

Schema I

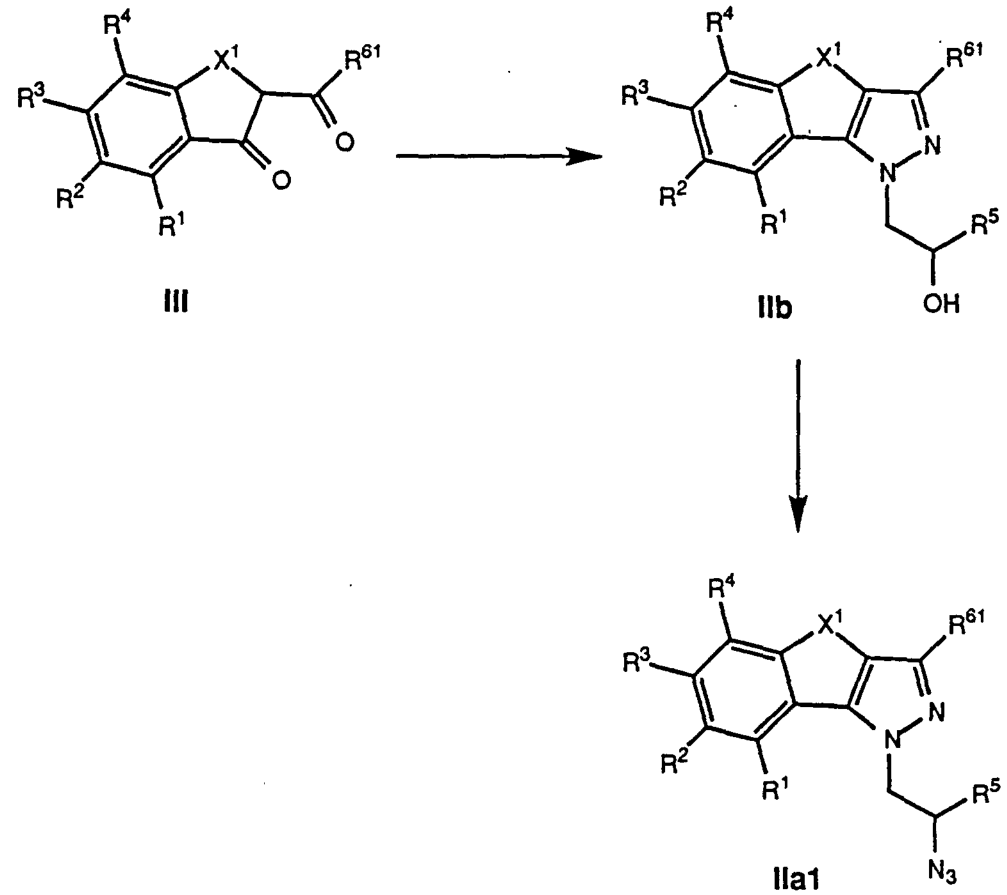

III

IIb

IIa1

Schema 2

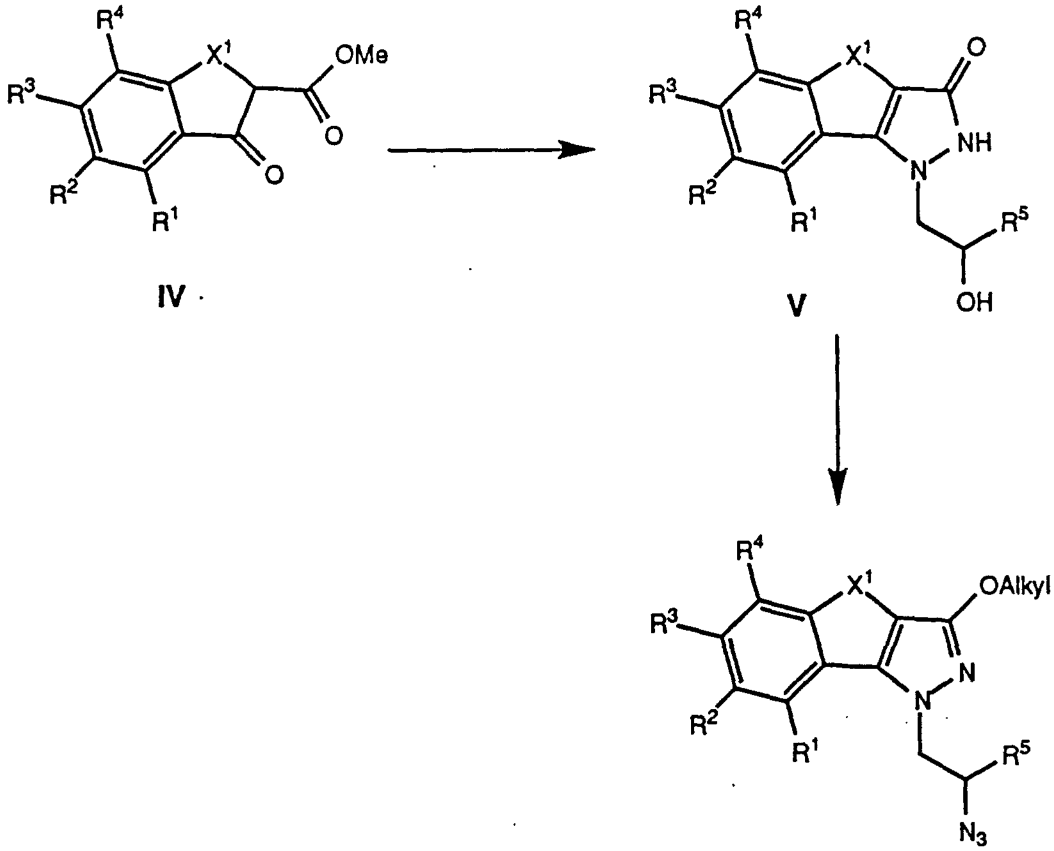

IV

V

IIa2

Das Reaktionsschema 1 zeigt die Herstellung von Verbindungen der Formel IIa1, worin $R^{61}$ Wasserstoff oder niederes Alkyl bedeutet und alle anderen Substituenten die oben genannte Bedeutung haben, mit Ausnahme von X = -CH=CH-.

Zweckmässigerweise geht man dabei folgenderweise vor:

Eine bekannte oder durch bekannte Verfahren herstellbare Verbindung der Formel III wird mit 1-Hydrazino-2-propanol und p-Toluolsulfonsäure in wasserfreiem Toluol am Wasserabscheider zu der entsprechenden Pyrazolverbindung der allgemeinen Formel IIb1 umgesetzt. Anschliessend kann die Hydroxygruppe nach an sich bekannten Methoden in eine Abgangsgruppe überführt werden, beispielsweise durch Umsetzung mit einem Sulfonsäurechlorid, vorzugsweise Methansulfonsäurechlorid, zum Sulfonat. Durch Behandlung mit einem Azid, vorzugsweise mit Natriumazid, in einem polaren Lösungsmittel, z.B. DMF, können Verbindungen der Formel IIb1 in die entsprechenden Azidoverbindungen der Formel IIa1 umgesetzt werden, die, wie beschrieben, durch Reduktion der Azidgruppe in die erfindungsgemässen Verbindungen der Formel I überführt werden können.

Das Reaktionsschema 2 zeigt die Herstellung von Verbindungen der allgemeinen Formel IIa2, worin die Substituenten $R^1$ bis $R^5$ und X die oben beschriebene Bedeutung haben, mit Ausnahme von X = -CH=CH-.

Dabei wird zweckmässigerweise eine Verbindung der Formel IV, die in der Literatur bekannt ist oder durch bekannte Verfahren hergestellt werden kann, wie oben beschrieben mit 1-Hydrazino-2-propanol zu einer Verbindung der Formel V umgesetzt. Anschliessend wird diese Verbindung in einem wasserfreien Lösungsmittel alkyliert. Als Alkylierungsmittel können bevorzugt Dialkylsulfate oder Diazomethan verwendet werden. Anschliessend kann die OH-Gruppe der Verbindung V nach oben geschilderten Methoden in eine Abgangsgruppe überführt und dann durch eine Azidgruppe ersetzt werden.

Das folgende Reaktionsschema 3 zeigt die Herstellung der Verbindungen der Formel Ib, worin die Substituenten $R^1$ bis $R^6$ die oben genannte Bedeutung haben.

Schema 3

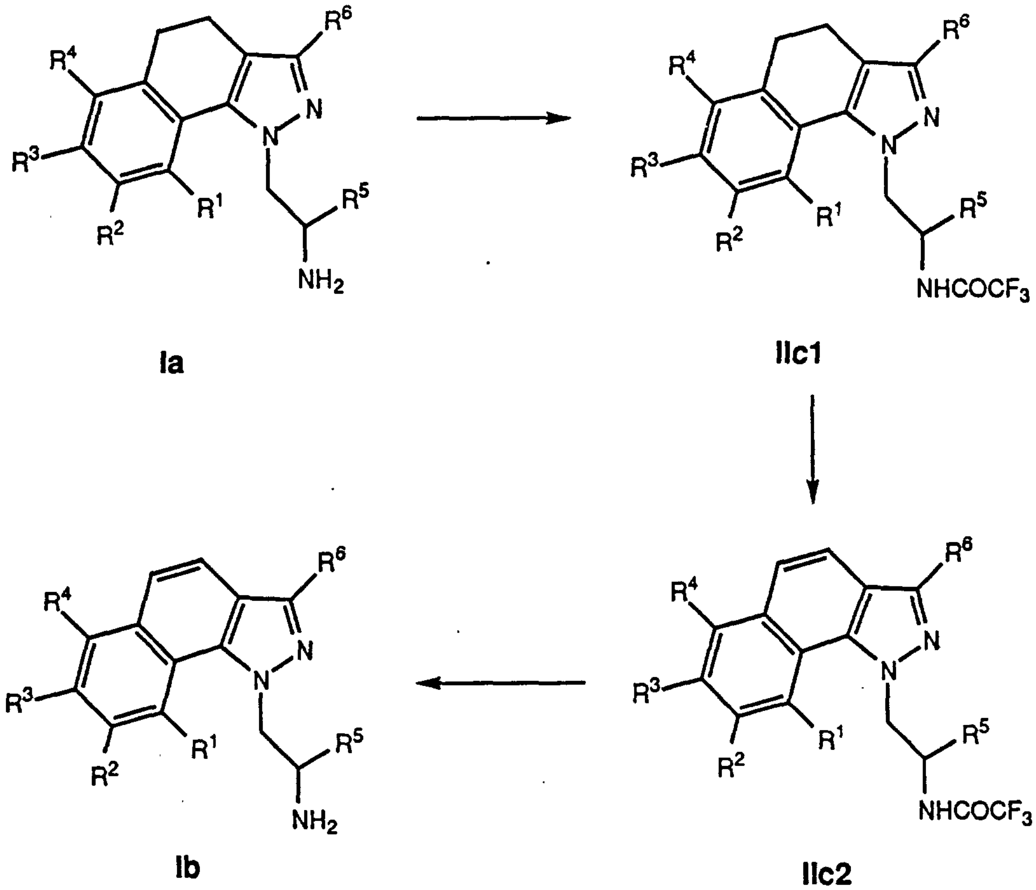

Ia IIc1

Ib IIc2

Zweckmässigerweise geht man wie folgt vor:

Eine Verbindung der Formel Ia wird in einer Lösung, bestehend aus Triethylamin und Trifluoressigsäureethylester in einem wasserfreien Lösungsmittel, vorzugsweise Methanol, umgesetzt. Nach Abzug des Lösungsmittels wird der Rückstand in Dioxan aufgenommen, mit DDQ (2,3-Dichlor-5,6-dicyan-1,4-benzochinon) versetzt und refluxiert. Nach dieser Dehydrierung kann die Schutzgruppe, wie beschrieben, von der Aminogruppe abgespalten werden. Bei dieser Umsetzung ist die Aminoschutzgruppe $-COCF_3$ besonders gut geeignet, aber auch andere Schutzgruppen können verwendet werden.

Wie eingangs erwähnt, besitzen die Verbindungen der allgemeinen Formel I und ihre pharmazeutisch annehmbaren Salze wertvolle pharmakologische Eigenschaften. Sie sind in der Lage, an Serotonin-Rezeptoren zu binden und eignen sich daher zur Behandlung oder Verhütung von Krankheiten oder Störungen der eingangs erwähnten Art bzw. zur Herstellung entsprechender Arzneimittel.

Die Bindung der erfindungsgemässen Verbindungen der Formel I an die Serotonin-Rezeptoren wurde durch Standardmethoden in vitro bestimmt. Die Präparate wurden gemäss den nachfolgend angegebenen Tests geprüft:

a) Zur Bestimmung der Affinität einer Verbindung zum $5HT_{1A}$-Rezeptor wurden Verdrängungsversuche mit [$^3$H]-5HT (1 nM) als Radioligand an rekombinierten human-$5HT_{1A}$ Rezeptoren, expremiert in 3T3-Zellen von Mäusen, durchgeführt. Es wurden Membranen verwendet, die von $2x10^5$ Zellen erhalten wurden. Die jeweilige Testverbindung wurde bei verschiedenen Konzentrationen untersucht.

b) Für die Bindung an den $5HT_{2C}$-Rezeptor gemäss dem [3H]-5HT-Bindungs-Test nach der Methode von S.J. Peroutka et al., Brain Research 584,191-196 (1992).

c) Für die Bindung an den $5HT_{2A}$-Rezeptor gemäss dem [3H]-DOB-Bindungs-Test nach der Methode von T. Branchek et. al., Molecular Pharmacology 38, 604-609 (1990).

Es sind die pki-Werte (pki = $-\log_{10}Ki$) der zu prüfenden Substanzen angegeben. Der ki-Wert ist durch folgende Formel definiert:

$$Ki = \frac{IC_{50}}{1 + \frac{[L]}{K_D}}$$

wobei die $IC_{50}$ - Werte diejenigen Konzentrationen der zu prüfenden Verbindungen in nM sind, durch welche 50% des am Rezeptor gebundenen Liganden verdrängt werden. [L] ist die Konzentration des Liganden und der $K_D$-Wert die Dissoziationskonstante des Liganden.

Die so ermittelte Aktivität einiger erfindungsgemässer Verbindungen ist aus nachfolgender Tabelle ersichtlich:

| **Testmethode** | | | |
|---|---|---|---|
| | **a** | **b** | **c** |
| 1 | 6.45 | 8.26 | 7.03 |
| 2 | 6.47 | 8.57 | 7.31 |
| 3 | 5.38 | 8.32 | 6.64 |
| 4 | 5.58 | 8.65 | 7.43 |
| 5 | 6.20 | 7.90 | 6.72 |
| 6 | 5.74 | 8.33 | 7.31 |
| 7 | 5.61 | 7.73 | 6.44 |
| 8 | 5.17 | 7.13 | 6.08 |
| 9 | 5.37 | 5.80 | 4.80 |
| 10 | 5.78 | 8.32 | 7.30 |
| 11 | 5.75 | 7.51 | 6.58 |
| 12 | 5.91 | 7.72 | 6.85 |
| 13 | 5.92 | 8.38 | 7.31 |
| 14 | 5,63 | 6.70 | 5.81 |
| 15 | 5,89 | 8.28 | 7.09 |
| 16 | 6,70 | 8.94 | 7.60 |
| 17 | | 7.40 | 6.68 |
| 18 | 6.00 | 8.48 | 7.31 |

1 = (RS)-2-(7-Methoxy-1,4-dihydro-indeno[2,1-c]pyrazol-1-yl)-1-methylethylamin fumarat (1:1)
2 = (S)-2-(7-Methoxy-1,4-dihydro-indeno[2,1-c]pyrazol-1-yl)-1-methylethylamin fumarat (1:1)
3 = (S)-2-(4,4,7-Trimethyl-1,4-dihydro-indeno[2,1-c]pyrazol-1-yl)-1-methylethylamin fumarat (1:1)
4 = (S)-2-(7-Methoxy-4,4-dimethyl-1,4-dihydro-indeno[2,1-c]pyrazol-1-yl)-1-methyl-ethylamin fumarat (1:1)
5 = (RS)-2-(4,4,7-Trimethyl-1,4-dihydro-indeno[2,1-c]pyrazol-1-yl)-1-methylethylamin fumarat (1:1)
6 = (RS)-2-(7-Methoxy-4,4-dimethyl-1,4-dihydro-indeno[2,1-c]pyrazol-1-yl)-1-methyl-ethylamin fumarat (1:1)
7 = (R)-2-(7-Methoxy-1,4-dihydro-indeno[2,1-c]pyrazol-1-yl)-1-methylethylamin fumarat (1:1)
8 = (RS)-2-(7-Methoxy-4-methyl-1,4-dihydro-indeno[2,1-c]pyrazol-1-yl)-1-methyl-ethylamin fumarat (1:1)
9 = (RS)-2-(3,7-Dimethoxy-1,4-dihydro-indeno[2,1-c]pyrazol-1-yl)-1-methylethylamin fumarat (1:0.5)
10 = (RS)-2-(7-Methyl-1,4-dihydro-indeno[2,1-c]pyrazol-1-yl)-1-methylethylamin fumarat (1:1)

(fortgesetzt)

| Testmethode | | | |
|---|---|---|---|
| | a | b | c |
| 11 = (RS)-2-(7-Fluor-1,4-dihydro-indeno[2,1-c]pyrazol-1-yl)-1-methylethylamin fumarat (1:1)<br>12 = (RS)-2-(7-Fluor-4,4-dimethyl-1,4-dihydro-indeno[2,1-c]pyrazol-1-yl)-1-methyl-ethylamin fumarat (1:1)<br>13 = (RS)-2-(7-Ethoxy-1,4-dihydro-indeno[2,1-c]pyrazol-1-yl)-1-methylethylamin fumarat (1:1)<br>14 = (RS)-2-(6-Methoxy-4-methyl-1,4-dihydro-indeno[2,1-c]pyrazol-1-yl)-1-methyl-ethylamin fumarat (1:1)<br>15 = (RS)-2-(8-Methoxy-4,5-dihydro-1H-benz[g]indazol-1-yl)-1-methylethylamin fumarat (1:1)<br>16 = (RS)-2-(8-Methoxy-1H-benz[g]indazol-1-yl)-1-methyl-ethylamin fumarat (1:0.5)<br>17 = (R)-2-(7-Ethoxy-1,4-dihydro-indeno[2,1-c]pyrazol-1-yl)-1-methylethylamin fumarat<br>18 = (S)-2-(7-Ethoxy-1,4-dihydro-indeno[2,1-c]pyrazol-1-yl)-1-methylethylamin fumarat | | | |

Peniserektionen (Ratten)

Es wurde gezeigt, dass die Peniserektion abhängig ist von der Stimulierung des $5HT_{2C}$ Rezeptors (vergl. Berendsen & Broekkamp, Eur. J. Pharmacol. 135, 179-184 (1987).

Innerhalb 45 Minuten nach Verabreichen der Testsubstanz an die Tiere wurde die Anzahl der Peniserektionen bestimmt. Die $ED_{50}$ ist die Dosis, die 50% dieser Erektionen hervorruft.

| Beispiel No. | $ED_{50}$ (mg/kg), s.c.) |
|---|---|
| 1 | 0.32 s.c. / 3.2 p.o. |
| 2 | 0.32 s.c. / 1.4 p.o. |
| 13 | 0.5 s.c. / 2.7 p.o. |
| 18 | 0.7 s.c. / 2.3 p.o. |

Die Verbindungen der Formel I und die pharmazeutisch annehmbaren Säureadditionssalze der Verbindungen der Formel I können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspension, verabreicht werden. Die Verabreichung kann aber auch rektal, z.B. in Form von Suppositorien, parenteral, z.B. in Form von Injektionslösungen, oder nasal erfolgen.

Zur Herstellung von pharmazeutischen Präparaten können die Verbindungen der Formel I und die pharmazeutisch annehmbaren Säureadditionssalze der Verbindungen der Formel I mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Glyzerin, pflanzliches Oel und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Arzneimittel, enthaltend eine Verbindung der Formel I oder ein pharmazeutisch annehmbares Säureadditionssalz davon und einen therapeutisch inerten Träger, sind ebenfalls Gegenstand der vorliegenden Erfindung, ebenso ein Verfahren zu deren Herstellung, das dadurch gekennzeichnet ist, dass man eine oder mehrere Verbindungen der Formel I und/oder pharmazeutisch annehmbare Säureadditionssalze wertvolle Stoffe zusammen mit einem oder mehreren therapeutisch inerten Trägern in eine galenische Darreichungsform bringt.

Erfindungsgemäss kann man Verbindungen der allgemeinen Formel I sowie deren pharmazeutisch annehmbare Säureadditionssalze bei der Behandlung oder Verhütung von zentralnervösen Störungen, wie Depressionen,

bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc.; Schädigungen des Nervensystems durch Trauma, Schlaganfall, neurodegenerative Erkrankungen etc.; cardiovasculären Störungen, wie Bluthochdruck, Thrombose, Schlaganfall etc.; und gastrointestinalen Störungen, wie Dysfunktion der Motilität des Gastrointestinaltrakts bzw. zur Herstellung entsprechender Arzneimittel verwenden. Die Dosierung kann innerhalb weiter Grenzen variieren und ist natürlich in jedem einzelnen Fall den individuellen Gegebenheiten anzupassen. Bei oraler Verabreichung liegt die Dosis in einem Bereich von etwa 0,01 mg pro Dosis bis etwa 500 mg pro Tag einer Verbindung der allgemeinen Formel I bzw. der entsprechenden Menge eines pharmazeutisch annehmbaren Säureadditionssalzes davon, wobei aber die obere Grenze auch überschritten werden kann, wenn sich dies als angezeigt erweisen sollte.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.
In den nachfolgenden Beispielen soll die vorliegende Erfindung weiter erläutert werden, ohne ihren Inhalt aber in irgend einer Weise einzuschränken.

__Beispiel 1__

a) Eine Lösung von 0.95 g (5 mmol) 2-Hydroxymethylen-6-methoxy-1-indanon, 0.55 g (6 mmol) (RS)-1-Hydrazino-2-propanol und 60 mg p-Toluolsulfonsäure in 60 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 0.9 g (74%) (RS)-1-(7-Methoxy-1,4-dihydro-indeno[2,1-c]pyrazol-1-yl)-propan-2-ol als gelbes Öl, das direkt in die nächste Reaktion eingesetzt wurde.

b) Zu einer auf 0° gekühlten Lösung von 0.9 g (3.7 mmol) (RS)-1-(7-Methoxy-1,4-dihydro-indeno[2,1-c]pyrazol-1-yl)-propan-2-ol und 2 ml (14.8 mmol) Triethylamin in 40 ml Dichlormethan gab man tropfenweise unter Rühren 0.6 ml (7.4 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 50 ml gesättigter Natriumhydrogencarbonatlösung gewaschen und die vereinigten Wasserphasen einmal mit 50 ml Dichlormethan extrahiert. Die vereinigten organischen Phasen wurden mit 50 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.48 g (7.4 mmol) Natriumazid versetzt und das Reaktionsgemisch unter Rühren 15 Stunden auf 70° erwärmt. Nach dem Abkühlen goss man die Lösung auf 80 ml halbgesättigte Natriumchloridlösung und extrahierte zweimal mit jeweils 80 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 0.87 g (87%) (RS)-1-(2-Azidopropyl)-7-methoxy-1,4-dihydro-indeno[2,1-c]pyrazol als schwach gelbes Öl.

c) 0.85 g (3.2 mmol) (RS)-1-(2-Azido-propyl)-7-methoxy-1,4-dihydroindeno-[2,1-c]-pyrazol gelöst in 50 ml wasserfreiem Ethanol wurden an 85 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 371 mg (3.2 mmol) Fumarsäure in 10 ml Methanol versetzt. Man rührte 15 Stunden bei Raumtemperatur und filtrierte anschliessend die weissen Kristalle ab. Man erhielt 0.9 g (78%) (RS)-2-(7-Methoxy-1,4-dihydro-indeno[2,1-c]pyrazol-1-yl)-1-methylethylamin fumarat (1:1) mit Smp. 182°.

__Beispiel 2__

a) Eine Lösung von 1.5 g (7.9 mmol) 2-Hydroxymethylen-6-methoxy-1-indanon, 0.78 g (8.6 mmol) (R)-1-Hydrazino-2-propanol und 100 mg p-Toluolsulfonsäure in 100 ml wasserfreiem Toluol wurde 1.5 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 1.3 g (68%) (R)-1-(7-Methoxy-1,4-dihydro-indeno[2,1-c]pyrazol-1-yl)-propan-2-ol als gelben Feststoff, der direkt in die nächste Reaktion eingesetzt wurde.

b) Zu einer auf 0° gekühlten Lösung von 1.3 g (5.3 mmol) (R)-1-(7-Methoxy-1,4-dihydro-indeno[2,1-c]pyrazol-1-yl)-propan-2-ol und 3.05 ml (21.4 mmol) Triethylamin in 50 ml Dichlormethan gab man tropfenweise unter Rühren 0.85 ml (10.7 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.83 g (12.5 mmol) Natriumazid versetzt und das Reaktionsgemisch unter Rühren 15 Stunden auf 70° erwärmt. Nach dem Abkühlen goss man die Lösung auf 100 ml halbgesättigte Natriumchloridlösung und extrahierte zweimal mit jeweils 100 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1.3 g (90%) (S)-1-(2-Azido-propyl)-7-methoxy-1,4-dihydro-indeno [2,1-c]pyrazol als schwach gelbes Öl.

c) 1.3 g (4.8 mmol) (S)-1-(2-Azido-propyl)-7-methoxy-1,4-dihydro-indeno-[2,1-c]-pyrazol gelöst in 50 ml wasserfreiem Ethanol wurden an 130 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 560 mg (4.8 mmol) Fumarsäure in 10 ml Methanol versetzt. Man rührte 4 Stunden bei Raumtemperatur und filtriertte anschliessend die weissen Kristalle ab. Man erhielt 1.4 g (81%) (S)-2-(7-Methoxy-1,4-dihydro-indeno[2,1-c]pyrazol-1-yl)-1-methyl-ethylamin fumarat (1:1) mit Smp. 180°.

**Beispiel 3**

a) Eine Lösung von 0.7 g (3.5 mmol) 2-Hydroxymethylen-3,3,6-trimethyl-1-indanon, 0.37 g (4.1 mmol) (R)-1-Hydrazino-2-propanol und 50 mg p-Toluolsulfonsäure in 50 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester) gereinigt. Man erhielt 0.8 g (89%) (R)-1-(4,4,7-Trimethyl-1,4-dihydroindeno[2,1-c]pyrazol-1-yl)-propan-2-ol als gelbes Öl, das direkt in die nächste Reaktion eingesetzt wurde.

b) Zu einer auf 0° gekühlten Lösung von 0.8 g (3.1 mmol) (R)-1-(4,4,7-Trimethyl-1,4-dihydro-indeno[2,1-c]pyrazol-1-yl)-propan-2-ol und 1.75 ml (12.5 mmol) Triethylamin in 50 ml Dichlormethan gab man tropfenweise unter Rühren 0.5 ml (6.24 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.41 g (6.3 mmol) Natriumazid versetzt und das Reaktionsgemisch unter Rühren 15 Stunden auf 70° erwärmt. Nach dem Abkühlen goss man die Lösung auf 100 ml halbgesättigte Natriumchloridlösung und extrahierte zweimal mit jeweils 100 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 0.7 g (80%) (S)-1-(2-Azido-propyl)-4,4,7-trimethyl-1,4-dihydroindeno [2,1-c]pyrazol als schwach gelbes Öl.

c) 0.7 g (2.5 mmol) (S)-1-(2-Azido-propyl)-4,4,7-trimethyl-1,4-dihydroindeno-[2,1-c]-pyrazol gelöst in 50 ml wasserfreiem Ethanol wurden an 70 mg Platinoxid 2 Stunden hydriert. Es wurde anschliessend vom Katalysator abfiltriert mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 290 mg (2.5 mmol) Fumarsäure in 5 ml Methanol versetzt. Man rührte 4 Stunden bei Raumtemperatur und filtrierte anschliessend die weissen Kristalle ab. Man erhielt 0.5 g (54%) (S)-2-(4,4,7-Trimethyl-1,4-dihydro-indeno[2,1-c]pyrazol-1-yl)-1-methyl-ethylamin fumarat (1:1) mit Smp. 158°.

**Beispiel 4**

a) Eine Lösung von 1.5 g (6.8 mmol) 2-Hydroxymethylen-6-methoxy-3,3-dimethyl-1-indanon, 0.74 g (8.2 mmol) (R)-1-Hydrazino-2-propanol und 100 mg p-Toluolsulfonsäure in 100 ml wasserfreiem Toluol wurde 1.5 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 1.41 g (76%) (R)-1-(7-Methoxy-4,4-dimethyl-1,4-dihydro-indeno[2,1-c]pyrazol-1-yl)-propan-2-ol als gelbes Öl, das direkt in die nächste Reaktion eingesetzt wurde.

b) Zu einer auf 0° gekühlten Lösung von 1.41 g (5.2 mmol) (R)-1-(7-Methoxy-4,4-dimethyl-1,4-dihydro-indeno [2,1-c]pyrazol-1-yl)-propan-2-ol und 2.9 ml (20.4 mmol) Triethylamin in 50 ml Dichlormethan gab man tropfenweise unter Rühren 0.8 ml (10.2 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.76 g (11.4 mmol) Natriumazid versetzt und das Reaktionsgemisch unter Rühren 15 Stunden auf 70° erwärmt. Nach dem Abkühlen goss man die Lösung auf 100 ml halbgesättigte Natriumchloridlösung und extrahierte zweimal mit jeweils 100 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1.38 g (89%) (S)-1-(2-Azido-propyl)-7-methoxy-4,4-dimethyl-1,4-dihydro-indeno[2,1-c]pyrazol als gelbes Öl.

c) 1.38 g (4.6 mmol) (S)-1-(2-Azido-propyl)-7-methoxy-4,4-dimethyl-1,4-dihydro-indeno-[2,1-c]-pyrazol gelöst in 50 ml wasserfreiem Ethanol wurden an 140 mg Platinoxid 1.5 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 534 mg (4.6 mmol) Fumarsäure in 10 ml Methanol versetzt. Man rührte 18 Stunden bei Raumtemperatur und filtrierte anschliessend die weissen Kristalle ab. Man erhielt 1.23 g (69%) (S)-2-(7-Methoxy-4,4-dimethyl-1,4-dihydro-indeno[2,1-c]pyrazol-1-yl)-1-methyl-ethylamin fumarat (1:1) mit Smp. 160-1620.

**Beispiel 5**

a) Eine Lösung von 1.5 g (7.4 mmol) 2-Hydroxymethylen-3,3,6-trimethyl-1-indanon, 0.55 g (6.1 mmol) (RS)-1-Hydrazino-2-propanol und 100 mg p-Toluolsulfonsäure in 100 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 1.6 g (84%) (RS)-1-(4,4,7-Trimethyl-1,4-dihydro-indeno [2,1-c]pyrazol-1-yl)-propan-2-ol als gelbes Öl, das direkt in die nächste Reaktion eingesetzt wurde.

b) Zu einer auf 0° gekühlten Lösung von 1.6 g (6.2 mmol) (RS)-1-(4,4,7-Trimethyl-1,4-dihydro-indeno[2,1-c]pyrazol-1-yl)-propan-2-ol und 3.5 ml (25 mmol) Triethylamin in 60 ml Dichlormethan gab man tropfenweise unter Rühren 1 ml (12.5 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 60 ml wasserfreiem Dimethylformamid gelöst, mit 0.81 g (12.5 mmol) Natriumazid versetzt und das Reaktionsgemisch unter Rühren 15 Stunden auf 70° erwärmt. Nach dem Abkühlen goss man die Lösung auf 100 ml halbgesättigte Natriumchloridlösung und extrahierte zweimal mit jeweils 100 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene gelbe Öl wurde durch Säulenchromatographie an Kieselgel (Essigester/Toluol 1:1) gereinigt. Man erhielt 1.1 g (63%) (RS)-1-(2-Azido-propyl)-4,4,7-trimethyl-1,4-dihydroindeno [2,1-c]pyrazol als schwach gelbes Öl.

c) 1.1 g (3.9 mmol) (RS)-1-(2-Azido-propyl)-4,4,7-trimethyl-1,4-dihydroindeno-[2,1-c]-pyrazol gelöst in 60 ml wasserfreiem Ethanol wurden an 110 mg Platinoxid 3 Stunden hydriert. Es wurde anschliessend vom Katalysator abfiltriert mit Ethanol nachgewaschen und das Lösungsmittel im Vakuum abgezogen. Das erhaltene farblose Öl wurde in 150 ml wasserfreiem Diethylether gelöst, filtriert und unter Rühren mit einer Lösung von 453 mg (3.9 mmol) Fumarsäure in 10 ml Methanol versetzt. Man rührte 4 Stunden bei Raumtemperatur und filtrierte anschliessend die weissen Kristalle ab. Man erhielt 1 g (69%) (RS)-2-(4,4,7-Trimethyl-1,4-dihydro-indeno[2,1-c]pyrazol-1-yl)-1-methyl-ethylamin fumarat (1:1) mit Smp. 167°.

**Beispiel 6**

a) Eine Lösung von 1.5 g (6.8 mmol) 2-Hydroxymethylen-6-methoxy-3,3-dimethyl-1-indanon, 0.74 g (8.2 mmol) (RS)-1-Hydrazino-2-propanol und 100 mg p-Toluolsulfonsäure in 100 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 1.4 g (75%) (RS)-1-(7-Methoxy-4,4-dimethyl-1,4-dihydro-indeno[2,1-c]pyrazol-1-yl)-propan-2-ol als gelbes Öl, das direkt in die nächste Reaktion eingesetzt wurde.

b) Zu einer auf 0° gekühlten Lösung von 1.4 g (5.1 mmol) (RS)-1-(7-Methoxy-4,4-dimethyl-1,4-dihydro-indeno [2,1-c]pyrazol-1-yl)-propan-2-ol und 2.9 ml (20.4 mmol) Triethylamin in 50 ml Dichlormethan gab man tropfenweise unter Rühren 0.8 ml (10.2 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.66 g (10.2 mmol) Natriumazid versetzt und das Reaktionsgemisch unter Rühren 15 Stunden auf 70° erwärmt. Nach dem Abkühlen goss man die Lösung auf 100 ml halbgesättigte Natriumchloridlösung und extrahierte zweimal mit jeweils 100 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1.03 g (68%) (RS)-1-(2-Azido-propyl)-7-methoxy-4,4-dimethyl-1,4-dihydro-indeno[2,1-c]pyrazol als gelbes Öl.

c) 1.03 g (3.5 mmol) (RS)-1-(2-Azido-propyl)-7-methoxy-4,4-dimethyl-1,4-dihydro-indeno-[2,1-c]-pyrazol gelöst in 50 ml wasserfreiem Ethanol wurden an 100 mg Platinoxid 1.5 Stunden hydriert. Es wurde anschliessend vom Katalysator abfiltriert, mit Ethanol nachgewaschen und das Lösungsmittel im Vakuum abgezogen. Das erhaltene gelbe Öl wurde in 80 ml wasserfreiem Diethylether gelöst, filtriert und unter Rühren mit einer Lösung von 440 mg (3.5 mmol) Fumarsäure in 15 ml Methanol versetzt. Man rührte 15 Stunden bei Raumtemperatur und filtrierte anschliessend die weissen Kristalle ab. Man erhielt 0.72 g (53%) (RS)-2-(7-Methoxy-4,4-dimethyl-1,4-dihydro-indeno[2,1-c]pyrazol-1-yl)-1-methyl-ethylamin fumarat (1:1) mit Smp. 178-180°.

**Beispiel 7**

a) Eine Lösung von 0.51 g (2.7 mmol) 2-Hydroxymethylen-6-methoxy-1-indanon, 0.29 g (3.2 mmol) (S)-1-Hydrazino-2-propanol und 50 mg p-Toluolsulfonsäure in 50 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester) gereinigt. Man erhielt 0.6 g (92%) (S)-1-(7-Methoxy-1,4-dihydro-indeno[2,1-c]pyrazol-1-yl)-propan-2-ol als gelben Feststoff, der direkt in die nächste Reaktion eingesetzt wurde.

b) Zu einer auf 0° gekühlten Lösung von 0.6 g (2.46 mmol) (S)-1-(7-Methoxy-1,4-dihydro-indeno[2,1-c]pyrazol-1-yl)-propan-2-ol und 1.4 ml (9.84 mmol) Triethylamin in 60 ml Dichlormethan gab man tropfenweise unter Rühren 0.4 ml (4.92 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 50 ml gesättigter Natriumhydrogencarbonatlösung gewaschen und die vereinigten Wasserphasen einmal mit 50 ml Dichlormethan extrahiert. Die vereinigten organischen Phasen wurden mit 50 ml gesättigter Natriumchloridlösung gewaschen, über

Magnesiumsulfat getrocknet und im Vakuum eingedampft. Das erhaltene braune Öl wurde in 40 ml wasserfreiem Dimethylformamid gelöst, mit 0.32 g (4.92 mmol) Natriumazid versetzt und das Reaktionsgemisch unter Rühren 15 Stunden auf 80° erwärmt. Nach dem Abkühlen goss man die Lösung auf 80 ml halbgesättigte Natriumchloridlösung und extrahierte zweimal mit jeweils 80 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 0.5 g (75%) (R)-1-(2-Azidopropyl)-7-methoxy-1,4-dihydro-indeno [2,1-c]pyrazol als gelbes Öl.

c) 0.5 g (1.85 mmol) (R)-1-(2-Azido-propyl)-7-methoxy-1,4-dihydroindeno-[2,1-c]-pyrazol gelöst in 50 ml wasserfreiem Ethanol wurden an 50 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 215 mg (1.85 mmol) Fumarsäure in 5 ml Methanol versetzt. Man rührte 15 Stunden bei Raumtemperatur und filtrierte anschliessend die weissen Kristalle ab. Man erhielt 0.55 g (83%) (R)-2-(7-Methoxy-1,4-dihydro-indeno[2,1-c]pyrazol-1-yl)-1-methylethylamin fumarat (1:1) mit Smp. 180°.

**Beispiel 8**

a) Eine Lösung von 0.9 g (4.41 mmol) 2-Acetyl-6-methoxy-1-indanon, 0.51 g (5.73 mmol) (RS)-1-Hydrazino-2-propanol und 70 mg p-Toluolsulfonsäure in 70 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester) gereinigt. Man erhielt 1.1 g (96%) (RS)-1-(7-Methoxy-3-methyl-1,4-dihydroindeno[2,1-c]pyrazol-1-yl)-propan-2-ol als gelben Feststoff, der direkt in die nächste Reaktion eingesetzt wurde.

b) Zu einer auf 0° gekühlten Lösung von 1.1 g (4.26 mmol) (RS)-1-(7-Methoxy-3-methyl-1,4-dihydro-indeno[2,1-c] pyrazol-1-yl)-propan-2-ol und 2.4 ml (17 mmol) Triethylamin in 60 ml Dichlormethan gab man tropfenweise unter Rühren 0.7 ml (8.52 mmol) Methansulfonylchlorid und rührte weitere 1.5 Stunden bei dieser Temperatur. Das Reaktionsgemisch wurde anschliessend mit 130 ml Dichlormethan verdünnt, zweimal mit jeweils 60 ml gesättigter Natriumhydrogencarbonatlösung gewaschen und die vereinigten Wasserphasen einmal mit 50 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene braune Öl wurde in 60 ml wasserfreiem Dimethylformamid gelöst, mit 0.55 g (8.46 mmol) Natriumazid versetzt und das Reaktionsgemisch unter Rühren 15 Stunden auf 80° erwärmt. Nach dem Abkühlen goss man die Lösung auf 80 ml halbgesättigte Natriumchloridlösung und extrahierte zweimal mit jeweils 80 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1 g (83%) (RS)-1-(2-Azidopropyl)-7-methoxy-3-methyl-1,4-dihydro-indeno[2,1-c]pyrazol als schwach braunes Öl.

c) 1.1 g (3.88 mmol) (RS)-1-(2-Azido-propyl)-7-methoxy-3-methyl-1,4-dihydro-indeno-[2,1-c]-pyrazol gelöst in 60 ml wasserfreiem Ethanol wurden an 110 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 120 ml wasserfreiem Diethylether gelöst, filtriert und unter Rühren mit einer Lösung von 450 mg (3.88 mmol) Fumarsäure in 5 ml Methanol versetzt. Man rührte 15 Stunden bei Raumtemperatur und filtrierte anschliessend die weissen Kristalle ab. Man erhielt 1.2 g (83%) (RS)-2-(7-Methoxy-4-methyl-1,4-dihydro-indeno[2,1-c]pyrazol-1-yl)-1-methyl-ethylamin fumarat (1:1) mit Smp. 184°.

**Beispiel 9**

a) Eine Lösung von 4.54 g (20.6 mmol) 2-Methoxycarbonyl-6-methoxy-1-indanon, 2.3 g (25.5 mmol) (RS)-1-Hydrazino-2-propanol und 150 mg p-Toluolsulfonsäure in 150 ml wasserfreiem Toluol wurde 4 Stunden am Wasserabscheider erhitzt. Nach dem Einengen im Vakuum wurde das Reaktionsgemisch mit Ethanol aufgenommen und der ausgeschiedene Feststoff abfiltriert. Das Filtrat wurde eingeengt und durch Säulenchromatographie an Kieselgel (Dichlormethan/Methanol 9:1) gereinigt. Man erhielt 2.44 g (46%) (RS)-1-(7-Methoxy-1,4-dihydro-indeno [2,1-c]pyrazol-3-on-1-yl)-propan-2-ol als braunes Öl, das direkt in die nächste Reaktion eingesetzt wurde.

b) Zu einer Lösung von 2.44 g (9.37 mmol) (RS)-1-(7-Methoxy-1,4-dihydro-indeno[2,1-c]pyrazol-3-on-1-yl)-propan-2-ol in 80 ml wasserfreiem Diethylether und 50 ml wasserfreiem Methanol gab man unter Rühren eine Lösung von 0.79 g (18.8 mmol) Diazomethan in 56 ml wasserfreiem Diethylether. Es wurde weitere 15 Stunden bei Raumtemperatur geührt und anschliessend im Vakuum eingeengt. Man erhielt 2.08 g (81%) (RS)-1-(3,7-Dimethoxy-1,4-dihydro-indeno[2,1-c]pyrazol-1-yl)-propan-2-ol als braunen Feststoff, der direkt in die nächste Reaktion eingesetzt wurde.

c) Zu einer auf 0° gekühlten Lösung von 2.08 g (7.6 mmol) (RS)-1-(3,7-Dimethoxy-1,4-dihydro-indeno[2,1-c]pyrazol-1-yl)-propan-2-ol und 4.3 ml (30.4 mmol) Triethylamin in 60 ml Dichlormethan gab man tropfenweise unter Rühren 1.21 ml (15.2 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 100 ml gesättigter Natriumhydrogencarbonatlösung gewaschen und die vereinigten Wasserphasen einmal mit 70 ml Dichlormethan extrahiert. Die vereinigten organischen Phasen wurden mit 100 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 1 g (15.4 mmol) Natriumazid versetzt und das Reaktionsgemisch unter Rühren 20 Stunden auf 70° erwärmt. Nach dem Abkühlen goss man die Lösung auf 80 ml halbgesättigte Natriumchloridlösung und extrahierte zweimal mit jeweils 80 ml Diethylether. Die vereinigten organischen Phasen wusch man je einmal mit 80 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1.11 g (49%) (RS)-1-(2-Azidopropyl)-3,7-dimethoxy-1,4-dihydro-indeno[2,1-c]pyrazol als braunes Öl.

d) 1.11 g (3.71 mmol) (RS)-1-(2-Azido-propyl)-3,7-dimethoxy-1,4-dihydroindeno-[2,1-c]-pyrazol gelöst in 60 ml wasserfreiem Ethanol wurden an 110 mg Platinoxid 1.5 Stunden hydriert. Es wurde anschliessend vom Katalysator abfiltriert, mit Ethanol nachgewaschen und das Lösungsmittel im Vakuum abgezogen. Das erhaltene farblose Öl wurde in 50 ml wasserfreiem Diethylether gelöst, filtriert und unter Rühren mit einer Lösung von 430 mg (3.71 mmol) Fumarsäure in 5 ml Methanol versetzt. Man rührte 15 Stunden bei Raumtemperatur und filtrierte anschliessend die beigen Kristalle ab. Man erhielt 0.56 g (46%) (RS)-2-(3,7-Dimethoxy-1,4-dihydro-indeno[2,1-c]pyrazol-1-yl)-1-methyl-ethylamin fumarat (1:0.5) mit Smp. 209°.

**Beispiel 10**

a) Eine Lösung von 1.4 g (8.04 mmol) 2-Hydroxymethylen-6-methyl-1-indanon, 0.87 g (9.65 mmol) (RS)-1-Hydrazino-2-propanol und 100 mg p-Toluolsulfonsäure in 100 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 1.7 g (93%) (RS)-1-(7-Methyl-1,4-dihydro-indeno[2,1-c] pyrazol-1-yl)-propan-2-ol als gelbes Öl, das direkt in die nächste Reaktion eingesetzt wurde.

b) Zu einer auf 0° gekühlten Lösung von 1.7 g (7.45 mmol) (RS)-1-(7-Methyl-1,4-dihydro-indeno[2,1-c]pyrazol-1-yl)-propan-2-ol und 4.12 ml (29.7 mmol) Triethylamin in 60 ml Dichlormethan gab man tropfenweise unter Rühren 1.15 ml (14.8 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 50 ml gesättigter Natriumhydrogencarbonatlösung gewaschen und die vereinigten Wasserphasen einmal mit 50 ml Dichlormethan extrahiert. Die vereinigten organischen Phasen wurden mit 50 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.96 g (14.8 mmol) Natriumazid versetzt und das Reaktionsgemisch unter Rühren 15 Stunden auf 70° erwärmt. Nach dem Abkühlen goss man die Lösung auf 100 ml halbgesättigte Natriumchloridlösung und extrahierte zweimal mit jeweils 100 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1.38 g (73%) (RS)-1-(2-Azidopropyl)-7-methyl-1,4-dihydro-indeno [2,1-c]pyrazol als schwach gelben Feststoff mit Smp. 70-72°.

c) 1.38 g (5.45 mmol) (RS)-1-(2-Azido-propyl)-7-methyl-1,4-dihydroindeno-[2,1-c]-pyrazol gelöst in 60 ml wasserfreiem Ethanol wurden an 140 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 633 mg (5.45 mmol) Fumarsäure in 10 ml Methanol versetzt. Man rührte 15 Stunden bei Raumtemperatur und filtrierte anschliessend die weissen Kristalle ab. Man erhielt 1.62 g (87%) (RS)-2-(7-Methyl-1,4-dihydroindeno[2,1-c]pyrazol-1-yl)-1-methyl-ethylamin fumarat (1:1) mit Smp. 205°.

**Beispiel 11**

a) Eine Lösung von 1.42 g (8.0 mmol) 6-Fluor-2-hydroxymethylen-1-indanon, 0.87 g (9.65 mmol) (RS)-1-Hydrazino-2-propanol und 100 mg p-Toluolsulfonsäure in 100 ml wasserfreiem Toluol wurde 1.5 Stunden am Wasserabscheider erhitzt. Nach dem Einengen im Vakuum wurde das Reaktionsgemisch durch Säulenchromatographie an Kieselgel (Essigester) gereinigt. Man erhielt 1.5 g (81%) (RS)-1-(7-Fluor-1,4-dihydro-indeno[2,1-c]pyrazol-1-yl)-propan-2-ol als gelben Feststoff, der direkt in die nächste Reaktion eingesetzt wurde.

b) Zu einer auf 0° gekühlten Lösung von 1.5 g (6.46 mmol) (RS)-1-(7-Fluor-1,4-dihydro-indeno[2,1-c]pyrazol-1-yl)-propan-2-ol und 3.6 ml (25.8 mmol) Triethylamin in 60 ml Dichlormethan gab man tropfenweise unter Rühren 1 ml (12.9 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 50 ml gesättigter Natriumhydrogencarbonatlösung gewaschen und die vereinigten Wasserphasen einmal mit 50 ml Dichlormethan extrahiert. Die vereinigten organischen Phasen wurden mit 50 ml gesättigter Natriumchloridlösung gewaschen, über Magnesiumsulfat getrocknet und im Vakuum eingedampft. Der erhaltene gelbe Feststoff wurde in 50 ml wasserfreiem Dimethylformamid gelöst, mit 0.84 g (12.9 mmol) Natriumazid versetzt und das Reaktionsgemisch unter Rühren 5 Stunden auf 90° erwärmt. Nach dem Abkühlen goss man die Lösung auf 70 ml halbgesättigte Natriumchloridlösung und extrahierte zweimal mit jeweils 100 ml Essigester. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester) gereinigt. Man erhielt 1.59 g (96%) (RS)-1-(2-Azido-propyl)-7-fluor-1,4-dihydro-indeno[2,1-c]pyrazol als schwach gelbes Öl.

c) 1.59 g (6.18 mmol) (RS)-1-(2-Azido-propyl)-7-fluor-1,4-dihydro-indeno-[2,1-c]-pyrazol gelöst in 50 ml wasserfreiem Ethanol wurden an 160 mg Platinoxid 14 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 717 mg (6.18 mmol) Fumarsäure in 10 ml Methanol versetzt. Man rührte 5 Stunden bei Raumtemperatur und filtrierte anschliessend die weissen Kristalle ab. Man erhielt 1.68 g (78%) (RS)-2-(7-Fluor-1,4-dihydro-indeno[2,1-c]pyrazol-1-yl)-1-methylethylamin fumarat (1:1) mit Smp. 168-170°.

**Beispiel 12**

a) Eine Lösung von 1.4 g (6.8 mmol) 6-Fluor-2-hydroxymethylen-3,3-dimethyl-1-indanon, 0.74 g (8.2 mmol) (RS)-1-Hydrazino-2-propanol und 100 mg p-Toluolsulfonsäure in 100 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 1.7 g (96%) (RS)-1-(7-Fluor-4,4-dimethyl-1,4-dihydro-indeno[2,1-c]pyrazol-1-yl)-propan-2-ol als gelbes Öl, das direkt in die nächste Reaktion eingesetzt wurde.

b) Zu einer auf 0° gekühlten Lösung von 1.7 g (6.5 mmol) (RS)-1-(7-Fluor-4,4-dimethyl-1,4-dihydro-indeno[2,1-c]pyrazol-1-yl)-propan-2-ol und 3.6 ml (26 mmol) Triethylamin in 60 ml Dichlormethan gab man tropfenweise unter Rühren 1 ml (13 mmol) Methansulfonylchlorid und rührte weitere 1.5 Stunden bei dieser Temperatur. Das Reaktionsgemisch wurde anschliessend mit 150 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.85 g (13 mmol) Natriumazid versetzt und das Reaktionsgemisch unter Rühren 15 Stunden auf 70° erwärmt. Nach dem Abkühlen goss man die Lösung auf 100 ml halbgesättigte Natriumchloridlösung und extrahierte zweimal mit jeweils 100 ml Diethylether. Die vereinigten organischen Phasen wusch man je einmal mit 100 ml Wasser und mit 100 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1.66 g (90%) (RS)-1-(2-Azido-propyl)-7-fluor-4,4-dimethyl-1,4-dihydroindeno[2,1-c]pyrazol als gelbes Öl.

c) 1.66 g (5.82 mmol) (RS)-1-(2-Azido-propyl)-7-fluor-4,4-dimethyl-1,4-dihydro-indeno-[2,1-c]-pyrazol gelöst in 80 ml wasserfreiem Ethanol wurden an 160 mg Platinoxid 1.5 Stunden hydriert. Es wurde anschliessend vom Katalysator abfiltriert, mit Ethanol nachgewaschen und das Lösungsmittel im Vakuum abgezogen. Das erhaltene gelbe Öl wurde in 80 ml wasserfreiem Diethylether gelöst, filtriert und unter Rühren mit einer Lösung von 676 mg (5.82 mmol) Fumarsäure in 10 ml Methanol versetzt. Man rührte 15 Stunden bei Raumtemperatur und filtrierte anschliessend die weissen Kristalle ab. Man erhielt 1.81 g (83%) (RS)-2-(7-Fluor-4,4-dimethyl-1,4-dihydro-indeno[2,1-c]pyrazol-1-yl)-1-methyl-ethylamin fumarat (1:1) mit Smp. 144-146°.

**Beispiel 13**

a) Eine Lösung von 1.63 g (8 mmol) 2-Hydroxymethylen-6-ethoxy-1-indanon, 0.87 g (9.65 mmol) (RS)-1-Hydrazino-2-propanol und 100 mg p-Toluolsulfonsäure in 100 ml wasserfreiem Toluol wurde 1 Stunde am Wasserabscheider erhitzt. Nach dem Einengen im Vakuum wurde das Reaktionsgemisch durch Säulenchromatographie an Kieselgel (Essigester) gereinigt. Man erhielt 2 g (97%) (RS)-1-(7-Ethoxy-1,4-dihydro-indeno[2,1-c]pyrazol-1-yl)-propan-2-ol als gelben Feststoff, der direkt in die nächste Reaktion eingesetzt wurde.

b) Zu einer auf 0° gekühlten Lösung von 2 g (7.7 mmol) (RS)-1-(7-Ethoxy-1,4-dihydro-indeno[2,1-c]pyrazol-1-yl)-propan-2-ol und 4.3 ml (31 mmol) Triethylamin in 50 ml Dichlormethan gab man tropfenweise unter Rühren 1.2 ml (15.5 mmol) Methansulfonylchlorid und rührte weitere 50 Minuten 'bei dieser Temperatur. Das Reaktionsgemisch wurde anschliessend mit 130 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 50 ml wasserfreiem Dimethylformamid gelöst, mit 1 g (15.5 mmol) Natriumazid versetzt und das Reaktionsgemisch unter Rühren 15 Stunden auf 75° erwärmt. Nach dem Abkühlen goss man die Lösung auf 80 ml halbgesättigte Natriumchloridlösung und extrahierte zweimal mit jeweils 100 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester) gereinigt. Man erhielt 2.06 g (94%) (RS)-1-(2-Azido-propyl)-7-ethoxy-1,4-dihydro-indeno[2,1-c]pyrazol als schwach gelbes Öl.

c) 2.05 g (7.2 mmol) (RS)-1-(2-Azido-propyl)-7-ethoxy-1,4-dihydro-indeno-[2,1-c]-pyrazol gelöst in 50 ml wasserfreiem Ethanol wurden an 200 mg Platinoxid 1.5 Stunden hydriert. Es wurde anschliessend vom Katalysator abfiltriert, mit Ethanol nachgewaschen und das Lösungsmittel im Vakuum abgezogen. Das erhaltene farblose Öl wurde in 100 ml wasserfreiem Diethylether gelöst, filtriert und unter Rühren mit einer Lösung von 836 mg (7.2 mmol) Fumarsäure in 10 ml Methanol versetzt. Man rührte 15 Stunden bei Raumtemperatur und filtrierte anschliessend die weissen Kristalle ab. Man erhielt 2.35 g (87%) (RS)-2-(7-Ethoxy-1,4-dihydro-indeno[2,1-c]pyrazol-1-yl)-1-methyl-ethylamin fumarat (1:1) mit Smp. 191°.

**Beispiel 14**

a) Eine Lösung von 1.4 g (7.36 mmol) 2-Hydroxymethylen-5-methoxy-1-indanon, 0.8 g (8.83 mmol) (RS)-1-Hydrazino-2-propanol und 100 mg p-Toluolsulfonsäure in 100 ml wasserfreiem Toluol wurde 1.5 Stunden am Wasserabscheider erhitzt. Nach dem Einengen im Vakuum wurde das Reaktionsgemisch durch Säulenchromatographie an Kieselgel (Essigester/Hexan 4:1) gereinigt. Man erhielt 1.74 g (97%) (RS)-1-(6-Methoxy-1,4-dihydro-indeno[2,1-c]pyrazol-1-yl)-propan-2-ol als gelbes Öl, das direkt in die nächste Reaktion eingesetzt wurde.

b) Zu einer auf 0° gekühlten Lösung von 1.74 g (7.12 mmol) (RS)-1-(6-Methoxy-1,4-dihydro-indeno[2,1-c]pyrazol-1-yl)-propan-2-ol und 3.85 ml (28.5 mmol) Triethylamin in 60 ml Dichlormethan gab man tropfenweise unter Rühren 1.15 ml (14.2 mmol) Methansulfonylchlorid und rührte weitere 50 Minuten bei dieser Temperatur. Das Reaktionsgemisch wurde anschliessend mit 100 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Das erhaltene gelbe Öl wurde in 40 ml wasserfreiem Dimethylformamid gelöst, mit 0.92 g (14.2 mmol) Natriumazid versetzt und das Reaktionsgemisch unter Rühren 5 Stunden auf 90° erwärmt. Nach dem Abkühlen goss man die Lösung auf 80 ml halbgesättigte Natriumchloridlösung und extrahierte zweimal mit jeweils 100 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1.58 g (82%) (RS)-1-(2-Azidopropyl)-6-methoxy-1,4-dihydro-indeno[2,1-c]pyrazol als schwach gelbes Öl.

c) 1.58 g (5.86 mmol) (RS)-1-(2-Azido-propyl)-6-methoxy-1,4-dihydroindeno-[2,1-c]-pyrazol gelöst in 50 ml wasserfreiem Ethanol wurden an 160 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 80 ml wasserfreiem Diethylether gelöst, filtriert und unter Rühren mit einer Lösung von 680 mg (5.86 mmol) Fumarsäure in 10 ml Methanol versetzt. Man rührte 15 Stunden bei Raumtemperatur und filtrierte anschliessend die weissen Kristalle ab. Man erhielt 1.81 g (86%) (RS)-2-(6-Methoxy-1,4-dihydroindeno[2,1-c]pyrazol-1-yl)-1-methyl-ethylamin fumarat (1:1) mit Smp. 192-194°.

**Beispiel 15**

a) Eine Lösung von 1.63 g (7.98 mmol) 2-Hydroxymethylen-7-methoxy-1-tetralon, 0.87 g (9.65 mmol) (RS)-1-Hydrazino-2-propanol und 100 mg p-Toluolsulfonsäure in 100 ml wasserfreiem Toluol wurde 1.5 Stunden am Wasserabscheider erhitzt. Nach dem Einengen im Vakuum wurde das Reaktionsgemisch durch Säulenchromatographie an Kieselgel (Essigester/Hexan 1:1) gereinigt. Man erhielt 1.52 g (74%) (RS)-1-(4,5-Dihydro-8-methoxy-1H-benz[g]indazol-1-yl)-propan-2-ol als gelbes Öl, das direkt in die nächste Reaktion eingesetzt wurde.

b) Zu einer auf 0° gekühlten Lösung von 1.52 g (5.88 mmol) RS)-1-(4,5-Dihydro-8-methoxy-1H-benz[g]indazol-1-yl)-propan-2-ol und 3.27 ml (23.5 mmol) Triethylamin in 60 ml Dichlormethan gab man tropfenweise unter Rühren 0.89 ml (11.8 mmol) Methansulfonylchlorid und rührte weitere 1.5 Stunden bei dieser Temperatur. Das Reaktionsgemisch wurde anschliessend mit 100 ml Dichlormethan verdünnt, zweimal mit jeweils 50 ml gesättigter Natriumhydrogencarbonatlösung gewaschen und die vereinigten Wasserphasen einmal mit 50 ml Dichlormethan extrahiert. Die vereinigten organischen Phasen wurden mit 50 ml gesättigter Natriumchloridlösung gewaschen, über Magnesiumsulfat getrocknet und im Vakuum eingedampft. Das erhaltene braune Öl wurde in 50 ml wasserfreiem Dimethylformamid gelöst, mit 0.76 g (11.8 mmol) Natriumazid versetzt und das Reaktionsgemisch unter Rühren 15 Stunden auf 85° erwärmt. Nach dem Abkühlen goss man die Lösung auf 80 ml halbgesättigte Natriumchloridlösung und extrahierte zweimal mit jeweils 80 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene braune Öl wurde durch Säulenchromatographie an Kieselgel (Essigester/Toluol 1:1) gereinigt. Man erhielt 1 g (60%) (RS)-1-(2-Azidopropyl)-4,5-dihydro-8-methoxy-1H-benz[g]indazol als schwach gelbes Öl.

c) 1 g (3.5 mmol) (RS)-1-(2-Azido-propyl)-4,5-dihydro-8-methoxy-1H-benz[g]indazol gelöst in 50 ml wasserfreiem Ethanol wurden an 100 mg Platinoxid 2 Stunden hydriert. Es wurde anschliessend vom Katalysator abfiltriert, mit Ethanol nachgewaschen und das Lösungsmittel im Vakuum abgezogen. Das erhaltene farblose Öl wurde in 70 ml wasserfreiem Diethylether gelöst, filtriert und unter Rühren mit einer Lösung von 406 mg (3.5 mmol) Fumarsäure in 10 ml Methanol versetzt. Man rührte 15 Stunden bei Raumtemperatur und filtrierte anschliessend die weissen Kristalle ab. Man erhielt 0.98 g (75%) (RS)-2-(4,5-Dihydro-8-methoxy-1H-benz[g]indazol-1-yl)-1-methyl-ethylamin fumarat (1:1) mit Smp. 174-176°.

**Beispiel 16**

a) Eine Lösung von 0.86 g (3.34 mmol) (RS)-2-(4,5-Dihydro-8-methoxy-1H-benz[g]indazol-1-yl)-1-methyl-ethylamin, 0.56 ml (4 mmol) Triethylamin und 0.56 ml (4 mmol) Trifluoressigsäureethylester in 60 ml wasserfreiem Methanol wurde 50 Stunden bei Raumtemperatur gerührt. Nachdem man das Lösungsmittel im Vakuum abgezogen hatte, nahm man den Rückstand mit 70 ml wasserfreiem Dioxan auf, gab 0.8 g (3.5 mmol) DDQ hinzu und kochte 3 Stunden unter Rückfluss. Anschliessend engte man das Reaktionsgemisch im Vakuum ein und reinigte den Rückstand durch Säulenchromatographie an Kieselgel (Dichlormethan/Aceton 4:1). Man erhielt 0.97 g (82%) (RS)-N-[2-(8-Methoxy-1H-benz[g]indazol-1-yl)-1-methylethyl]-trifluoracetamid als hellbraunen Feststoff, der ohne weitere Umkristallisation in die nächste Reaktion eingesetzt wurde.

b) Eine Mischung aus 0.97 g (2.76 mmol) (RS)-N-[2-(8-Methoxy-1H-benz[g]indazol-1-yl)-1-methyl-ethyl]-trifluoracetamid 1 g (17.5 mmol) Kaliumhydroxid in 3 ml Wassser und 50 ml Methanol wurde 5 Stunden unter Rückfluss gekocht. Das Reaktionsgemisch wurde anschliessend in 100 ml 1N Natronlauge gegossen, dreimal mit jeweils 100 ml Diethylether extrahiert und die vereinigten organischen Phasen über Magnesiumsulfat getrocknet. Nach dem Einengen im Vakuum, wurde der Rückstand durch Säulenchromatographie an Kieselgel (Dichlormethan/Methanol 9:1) gereinigt. Man erhielt 0.62 g (2.43 mmol) gelbes Öl, das in 50 ml Diethylether gelöst und unter Rühren mit einer Lösung von 280 mg (2.43 mmol) Fumarsäure in 5 ml wasserfreiem Methanol versetzt wurde. Man rührte weitere 17 Stunden bei Raumtemperatur und filtrierte anschliessend die weissen Kristalle ab. Man erhielt 640 mg (74%) (RS)-2-(8-Methoxy-1H-benz[g]indazol-1-yl)-1-methyl-ethylamin fumarat (1:0.5) mit Smp. 196-198°C.

**Beispiel 17**

a) Eine Lösung von 1.6 g (7.83 mmol) 2-Hydroxymethylen-6-ethoxy-1-indanon, 0.85 g (9.40 mmol) (S)-1-Hydrazino-2-propanol und 70 mg p-Toluolsulfonsäure in 80 ml wasserfreiem Toluol wurde 2 Stunden am Wasserabscheider erhitzt. Nach dem Einengen im Vakuum wurde das Reaktionsgemisch durch Säulenchromatographie an Kieselgel (Essigester) gereinigt. Man erhielt 1.73 g (86%) (S)-1-(7-Ethoxy-1,4-dihydro-indeno[2,1-c]pyrazol-1-yl)-propan-2-ol als gelben Feststoff, der direkt in die nächste Reaktion eingesetzt wurde.

b) Zu einer auf 0° gekühlten Lösung von 1.73 g (6.7 mmol) (S)-1-Ethoxy-1,4-dihydro-indeno[2,1-c]pyrazol-1-yl)-propan-2-ol und 3.72 ml (26.8 mmol) Triethylamin in 50 ml Dichlormethan gab man tropfenweise unter Rühren 1.01 ml (13.4 mmol) Methansulfonylchlorid und rührte weitere 90 Minuten bei dieser Temperatur. Das Reaktionsgemisch wurde anschliessend mit 130 ml Dichlormethan verdünnt, zweimal mit jeweils 70 ml gesättigter Natriumhydrogencarbonatlösung gewaschen und die vereinigten Wasserphasen einmal mit 70 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Der erhaltene gelbliche Feststoff wurde in 50 ml wasserfreiem Dimethylformamid gelöst, mit 0.86 g (13.4 mmol) Natriumazid versetzt und das Reaktionsgemisch untr Rühren 16 Stunden auf 90° erwärmt. Nach dem Abkühlen goss man die Lösung auf 80 ml halbgesättigte Natriumchloridlösung und extrahierte zweimal mit jeweils 100 ml Diethylether. Die vereinigten organischen Phasen wusch man je einmal mit 80 ml Wasser und mit 80 ml gesättigter Natriumchloridlösung, trocknete über Magnesiumsulfat und engte die Lösung im Vakuum ein. Das erhaltene gelbe Oel wurde durch Säulenchromatographie an Kieselgel (Essigester) gereinigt. Man erhielt 1.76 g (93%) (R)-1-(2-Azido-propyl)-7-ethoxy-1,4-dihydro-indeno-[2,1-c]pyrazol als schwach gelben Feststoff.

c) 1.76 g (6.21 mmol) (R)-1-(2-Azido-propyl)-7-ethoxy-1,4-dihydro-indeno-[2,1-c]pyrazol gelöst in 100 ml wasserfreiem Ethanol wurden an 180 mg Platinoxid 17 Stunden hydriert. Es wurde anschliessend vom Katalysator abfiltriert, mit Ethanol nachgewaschen und das Lösungsmittel im Vakuum abgezogen. Das erhaltene farblose Oel wurde in 100 ml wasserfreiem Diethylether gelöst, filtriert und unter Rühren mit einer Lösung von 721 mg (6.21 mmol) Fumarsäure in 10 ml Methanol versetzt. Man rührte 15 Stunden bei Raumtemperatur und filtrierte anschliessend die weissen Kristalle ab. Man erhielt 2.0 g (86%) (R)-2-(7-Ethoxy-1,4-dihydro-indeno-[2,1-c]pyrazol-1-yl)-1-methyl-ethylamin fumarat (1:1) mit Smp. 161°.

**Beispiel 18**

a) Eine Lösung von 0.5 g (2.45 mmol) 2-Hydroxymethylen-6-ethoxy-1-indanon, 0.27 g (2.94 mmol) (R)-1-Hydrazino-2-propanol und 50 mg p-Toluolsulfonsäure in 50 ml wasserfreiem Toluol wurde 1 Stunde am Wasserabscheider erhitzt. Nach dem Einengen im Vakuum wurde das Reaktionsgemisch durch Säulenchromatographie an Kieselgel (Essigester) gereinigt. Man erhielt 0.49 g (77%) (R)-1-(7-Ethoxy-1,4-dihydro-indeno[2,1-c]pyrazol-1-yl)-propan-2-ol als gelben Feststoff, der direkt in die nächste Reaktion eingesetzt wurde.

b) Zu einer auf 0° gekühlten Lösung von 0.49 g (1.9 mmol) (R)-1-(7-Ethoxy-1,4-dihydro-indeno[2,1-c]pyrazol-1-yl)-propan-2-ol und 1.06 ml (7.6 mmol) Triethylamin in 30 ml Dichlormethan gab man tropfenweise unter Rühren 0.29 ml (3.79 mmol) Methansulfonylchlorid und rührte weitere 50 Minuten bei dieser Temperatur. Das Reaktionsgemisch wurde anschliessend mit 100 ml Dichlormethan verdünnt, zweimal mit jeweils 50 ml gesättigter Natriumhydrogencarbonatlösung gewaschen und die vereinigten Wasserphasen einmal mit 50 ml Dichlormethan extrahiert. Die vereinigten organischen Phasen wurden mit 70 ml gesättigter Natriumchloridlösung gewaschen, über Magnesiumsulfat getrocknet und im Vakuum eingedampft. Der erhaltene gelbe Feststoff wurde in 25 ml wasserfreiem Dimethylformamid gelöst, mit 0.25 g (3.8 mmol) Natriumazid versetzt und das Reaktionsgemisch unter Rühren 22 Stunden auf 70° erwärmt. Nach dem Abkühlen goss man die Lösung auf 70 ml halbgesättigte Nätriumchloridlösung und extrahierte zweimal mit jeweils 70 ml Diethylether. Die vereinigten organischen Phasen wusch man je einmal mit 50 ml Wasser und mit 50 ml gesättigter Natriumchloridlösung, trocknete über Magnesiumsulfat und engte die Lösung im Vakuum ein. Das erhaltene braune Oel wurde durch Säulenchromatographie an Kieselgel (Essigester) gereinigt. Man erhielt 0.53 g (99%) (S)-1-(2-Azido-propyl)-7-ethoxy-1,4-dihydro-indeno-[2,1-c]pyrazol als schwach gelben Feststoff.

c) 0.53 g (1.87 mmol) (S)-1-(2-Azido-propyl)-7-ethoxy-1,4-dihydro-indeno-[2,1-c]pyrazol gelöst in 25 ml wasserfreiem Ethanol wurden an 55 mg Platinoxid 1.5 Stunden hydriert. Es wurde anschliessend vom Katalysator abfiltriert, mit Ethanol nachgewaschen und das Lösungsmittel im Vakuum abgezogen. Das erhaltene farblose Oel wurde in 50 ml wasserfreiem Diethylether gelöst, filtriert und unter Rühren mit einer Lösung von 217 mg (1.87 mmol) Fumarsäure in 10 ml Methanol versetzt. Man rührte 15 Stunden bei Raumtemperatur und filtrierte anschliessend die weissen Kristalle ab. Man erhielt 0.54 g (77%) (S)-2-(7-Ethoxy-1,4-dihydro-indeno-[2,1-c]pyrazol-1-yl)-1-methyl-ethylamin fumarat (1:1) mit Smp. 157°.

**Beispiel A**

Es werden in üblicher Weise Tabletten folgender Zusammensetzung hergestellt:

| | mg/Tablette |
|---|---|
| Wirkstoff | 100 |
| Lactose pulv. | 95 |
| Maisstärke weiss | 35 |
| Polyvinylpyrrolidon | 8 |
| Na-carboxymethylstärke | 10 |
| Magnesiumstearat | 2 |
| | Tablettengewicht 250 |

**Beispiel B**

Es werden in üblicher Weise Tabletten folgender Zusammensetzung hergestellt:

| | mg/Tablette |
|---|---|
| Wirkstoff | 200 |
| Lactose pulv. | 100 |
| Maisstärke weiss | 64 |
| Polyvinylpyrrolidon | 12 |

(fortgesetzt)

| | mg/Tablette | |
|---|---|---|
| Na-carboxymethylstärke | | 20 |
| Magnesiumstearat | | 4 |
| | Tablettengewicht | 400 |

**Beispiel C**

Es werden Kapseln folgender Zusammensetzung hergestellt:

| | mg/Kapsel | |
|---|---|---|
| Wirkstoff | | 50 |
| Lactose krist. | | 60 |
| Mikrokristalline Cellulose | | 34 |
| Talk | | 5 |
| Magnesiumstearat | | 1 |
| | Kapselfüllgewicht | 150 |

Der Wirkstoff mit einer geeigneten Partikelgrösse, die Lactose kristallin und die mikrokristalline Cellulose werden homogen miteinander vermischt, gesiebt und danach Talk und Magnesiumstearat zugemischt. Die Endmischung wird in Hartgelatinekapseln geeigneter Grösse abgefüllt.

## Patentansprüche

**1.** Verbindungen der allgemeinen Formel

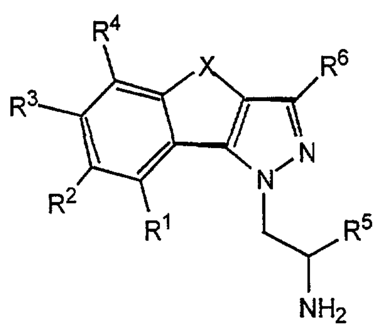

I

worin

| | |
|---|---|
| $R^1$ bis $R^4$ | je Wasserstoff, Hydroxy, Halogen, $(C_1$-$C_7)$-Alkyl, $(C_1$-$C_7)$-Alkoxy oder Phenyl; |
| $R^5$ | $(C_1$-$C_7)$-Alkyl; |
| $R^6$ | Wasserstoff, $(C_1$-$C_7)$-Alkyl oder $(C_1$-$C_7)$-Alkoxy; |
| X | $-(CR^7R^8)_n-$ oder -CH=CH-; |
| $R^7$ und $R^8$ | Wasserstoff oder $(C_1$-$C_7)$-Alkyl und |
| n | 1 oder 2 bedeuten, |

sowie pharmazeutisch annehmbare Salze von basischen Verbindungen der allgemeinen Formel I.

**2.** Verbindungen nach Anspruch 1, worin $R^5$ Methyl bedeutet.

**3.** Verbindungen nach Anspruch 1 oder 2, worin $R^2$ Methyl oder Methoxy, X $-CH_2-$ oder $-C(CH_3)_2$, $R^1$, $R^3$, $R^4$ und $R^6$ Wasserstoff bedeutet.

**4.** (RS)-2-(7-Methoxy-1,4-dihydro-indeno[2,1-c]pyrazol-1-yl)-1-methyl-ethylamin fumarat(1:1).

**5.** (S)-2-(7-Methoxy-1,4-dihydro-indeno[2,1-c]pyrazol-1-yl)-1-methylethylamin fumarat (1:1).

**6.** (S)-2-(4,4,7-Trimethyl-1,4-dihydro-indeno[2,1-c]pyrazol-1-yl)-1-methylethylamin fumarat (1:1).

**7.** (S)-2-(7-Methoxy-4,4-dimethyl-1,4-dihydro-indeno[2,1-c]pyrazol-1-yl)-1-methyl-ethylamin fumarat (1:1).

**8.** (RS)-2-(7-Methoxy-4,4-dimethyl-1,4-dihydro-indenol[2,1-c]pyrazol-1-yl)-1-methyl-ethylamin fumarat (1:1).

**9.** (RS)-2-(7-Ethoxy-1,4-dihydro-indeno[2,1-c]pyrazol-1-yl)-1-methylethylamin fumarat (1:1).

**10.** (R)-2-(7-Methoxy-1,4-dihydro-indeno[2,1-c]pyrazol-1-yl)-1-methylethylamin fumarat (1:1).

**11.** (RS)-2-(8-Methoxy-1-H-benz[g] indazol-1-yl)-1-methyl-ethylamin fumarat (1:0,5).

**12.** Verbindungen der allgemeinen Formel

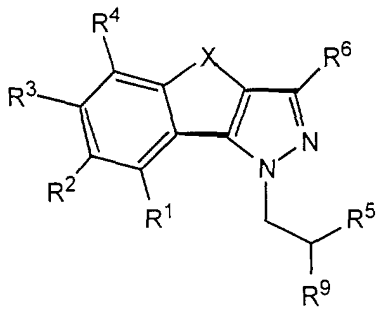

**II**

worin $R^1$ bis $R^6$ die in Anspruch 1 genannten Bedeutungen haben und $R^9$ eine Azidgruppe, eine Hydroxygruppe, eine Acetylaminogruppe oder eine Trifluoracetylaminogruppe bedeutet.

**13.** Arzneimittel, enthaltend eine Verbindung nach einem der Ansprüche 1-11 und ein therapeutisch inertes Trägermaterial, insbesondere zur Behandlung oder Verhütung von zentralnervösen Störungen, Depressionen, bipolaren Störungen, Angstzuständen, Schlaf- und Sexualstörungen, Psychosen, Schizophrenie, Migräne und anderen mit Kopfschmerz oder Schmerz anderer Art verbundenen Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc., Schädigungen des Nervensystems durch Trauma, Schlaganfall, neurodegenerative Erkrankungen etc., cardiovasculären Störungen, wie Bluthochdruck, Thrombose, Schlaganfall etc. und gastrointestinalen Störungen wie Dysfunktion der Motilität des Gastrointestinaltrakts.

**14.** Verfahren zur Herstellung von Verbindungen nach einem der Ansprüche 1 - 11, **dadurch gekennzeichnet, dass** man

a) eine Verbindung der allgemeinen Formel

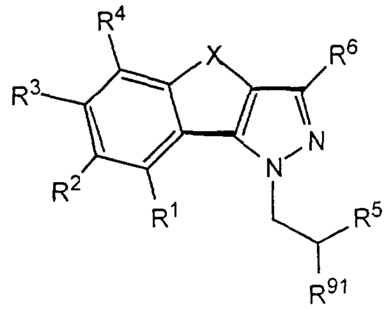

IIa

worin $R^1$ bis $R^6$ die in Anspruch 1 angegebene Bedeutung haben und $R^{91}$ eine Azidgruppe, eine Acetylaminogruppe oder eine Trifluoracetylaminogruppe bedeutet, in eine entsprechende Aminoverbindung der Formel 1 überführt und

b) erwünschtenfalls die erhaltene Verbindung der Formel I in ein pharmazeutisch annehmbares Salz überführt.

**15.** Verbindungen nach einem der Ansprüche 1 - 11 zur Anwendung als therapeutische Wirkstoffe, insbesondere zur Behandlung oder Verhütung von zentralnervösen Störungen, wie Depressionen, bipolaren Störungen, Angstzuständen, Schlaf- und Sexualstörungen, Psychosen, Schizophrenie, Migräne und andere mit Kopfschmerz oder Schmerz anderer Art verbundene Zustände, Persönlichkeitsstörungen oder obsessiv-compulsive Störungen, soziale Phobien oder panische Anfälle, mentale organische Störungen, mentale Störungen im Kindesalter, Aggressivität, altersbedingte Gedächtnisstörungen und Verhaltensstörungen, Sucht, Obesitas, Bulimie etc., Schädigungen des Nervensystems durch Trauma, Schlaganfall, neurodegenerative Erkrankungen etc., cardiovasculären Störungen, wie Bluthochdruck, Thrombose, Schlaganfall etc. und gastrointestinalen Störungen wie Dysfunktion der Motilität des Gastrointestinaltrakts.

**Claims**

**1.** Compounds of the general formula

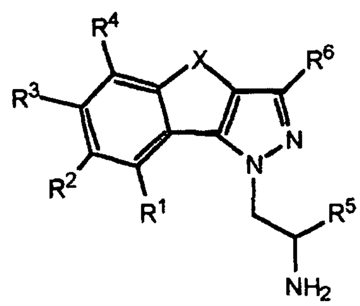

I

wherein

| | |
|---|---|
| $R^1$ to $R^4$ | each signify hydrogen, hydroxy, halogen, $(C_1\text{-}C_7)$-alkyl, $(C_1\text{-}C_7)$-alkoxy or phenyl; |
| $R^5$ | signifies $(C_1\text{-}C_7)$-alkyl; |
| $R^6$ | signifies hydrogen, $(C_1\text{-}C_7)$-alkyl or $(C_1\text{-}C_7)$-alkoxy; |
| X | signifies $-(CR^7R^8)_n-$ or -CH=CH-; |
| $R^7$ and $R^8$ | signify hydrogen or $(C_1\text{-}C_7)$-alkyl and |
| n | signifies 1 or 2, |

as well as pharmaceutically acceptable salts of basic compounds of general formula I.

**2.** Compounds according to claim 1, wherein $R^5$ signifies methyl.

**3.** Compounds according to claim 1 or 2, wherein $R^2$ signifies methyl or methoxy, X signifies $-CH_2-$ or $-C(CH_3)_2$, $R^1$, $R^3$, $R^4$ and $R^6$ signify hydrogen.

**4.** (RS)-2-(7-Methoxy-1,4-dihydro-indeno[2,1-c]pyrazol-1-yl)-1-methylethylamine fumarate (1:1).

**5.** (S)-2-(7-Methoxy-1,4-dihydro-indeno[2,1-c]pyrazol-1-yl)-1-methyl-ethylamine fumarate (1:1).

**6.** (S)-2-(4,4,7-Trimethyl-1,4-dihydro-indeno[2,1-c]pyrazol-1-yl)-1-methylethylamine fumarate (1:1).

**7.** (S)-2-(7-Methoxy-4,4-dimethyl-1,4-dihydro-indeno[2,1-c]pyrazol-1-yl)-1-methyl-ethylamine fumarate (1:1).

**8.** (RS)-2-(7-Methoxy-4,4-dimethyl-1,4-dihydro-indenol[2,1-c]pyrazol-1-yl)-1-methyl-ethylamine fumarate (1:1).

**9.** (RS)-2-(7-Ethoxy-1,4-dihydro-indeno[2,1-c]pyrazol-1-yl)-1-methyl-ethylamine fumarate (1:1).

**10.** (R)-2-(7-Methoxy-1,4-dihydro-indeno[2,1-c]pyrazol-1-yl)-1-methyl-ethylamine fumarate (1:1).

**11.** (RS)-2-(8-Methoxy-1-H-benz[g]indazol-1-yl)-1-methyl-ethylamine fumarate (1:0.5).

**12.** Compounds of the general formula

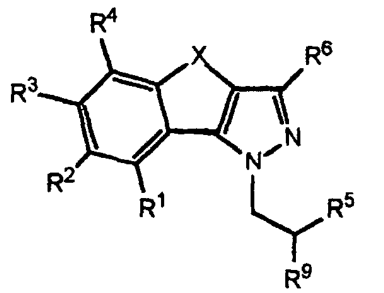

II

wherein $R^1$ to $R^6$ and X have the significances set forth in claim 1 and $R^9$ signifies an azido group, a hydroxy group, an acetylamino group or a trifluoroacetylamino group.

**13.** A medicament containing a compound according to any one of claims 1-11 and a therapeutically inert carrier material, especially for the treatment or prevention of central nervous disorders, depressions, bipolar disorders, anxiety states, sleep and sexual disorders, psychoses, schizophrenia, migraine and other conditions associated with cephalic pain or pain of a different kind, personality disorders or obsessive-compulsive disorders, social phobias or panic states, mental organic disorders, mental disorders in childhood, aggressivity, age-related memory disorders and behavioural disorders, addiction, obesity, bulimia etc., damages of the nervous system by trauma, stroke, neurodegenerative diseases etc., cardiovascular disorders such as hypertension, thrombosis, stroke etc. and gastrointestinal disorders such as dysfunction of the gastrointestinal tract motility.

**14.** A process for the manufacture of compounds according to any one of claims 1-11, **characterized by**

a) converting a compound of the general formula

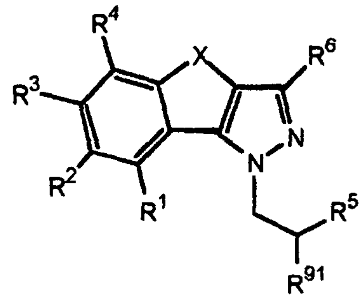

IIa

wherein $R^1$ to $R^6$ and X have the significance given in claim 1 and $R^{91}$ signifies an azido group, an acetylamino group or a trifluoroacetylamino group,
into a corresponding amino compound of formula I and

b) if desired, converting the compound of formula I obtained into a pharmaceutically acceptable salt

**15.** Compounds according to any one of claims 1 -11 for use as therapeutically active substances, especially for the treatment or prevention of central nervous disorders such as depressions, bipolar disorders, anxiety states, sleep and sexual disorders, psychoses, schizophrenia, migraine and other conditions associated with cephalic pain or pain of a different kind, personality disorders or obsessive-compulsive disorders, social phobias or panic states, mental organic disorders, mental disorders in childhood, aggressivity, age-related memory disorders and behavioural disorders, addiction, obesity, bulimia etc., damages of the nervous system by trauma, stroke, neurodegenerative diseases etc., cardiovascular disorders such as hypertension, thrombosis, stroke etc. and gastrointestinal disorders such as dysfunction of the gastrointestinal tract motility.

**Revendications**

**1.** Composés de formule générale :

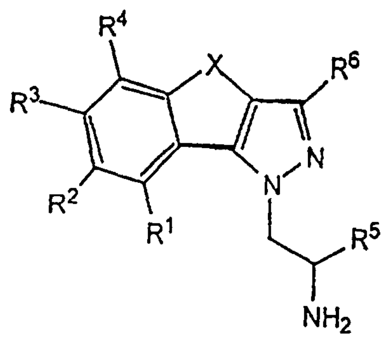

I

dans laquelle

- les radicaux $R^1$ à $R^4$ représentent chacun un atome d'hydrogène, un groupe hydroxy, halogéno, alkyle en $C_1$-$C_7$, alcoxy en $C_1$-$C_7$ ou phényle ;
- $R^5$ est un groupe alkyle en $C_1$-$C_7$ ;
- $R^6$ est un atome d'hydrogène ou un groupe alkyle en $C_1$-$C_7$ ou alcoxy en $C_1$-$C_7$;

- X est $-(CR^7R^8)_n-$ ou -CH=CH ;
- $R^7$ et $R^8$ sont des atomes d'hydrogène ou des groupes alkyle en $C_1$-$C_7$, et
- n vaut 1 ou 2,

ainsi que les sels acceptables d'un point de vue pharmaceutique de composés basiques de formule générale I.

**2.** Composés selon la revendication 1, dans lesquels $R^5$ est le groupe méthyle.

**3.** Composés selon la revendication 1 ou 2, dans lesquels $R^2$ est le groupe méthyle ou méthoxy, X est $-CH_2-$ ou $-C(CH_3)_2$, $R^1$, $R^3$, $R^4$ et $R^6$ sont des atomes d'hydrogène.

**4.** Fumarate (1:1) de la (RS)-2-(7-méthoxy-1,4-dihydroindéno[2,1-c] pyrazole-1-yl)-1-méthyléthylamine.

**5.** Fumarate (1:1) de la (S)-2-(7-méthoxy-1 ,4-dihydroindéno[2,1-c] pyrazole-1-yl)-1-méthyl-éthylamine.

**6.** Fumarate (1:1) de la (S)-2-(4,4,7-triméthyl-1,4-dihydroindéno[2,1-c] pyrazole-1-yl)-1-méthyléthylamine.

**7.** Fumarate (1:1) de la (S)-2-(7-méthoxy-4,4-diméthyl-1,4-indéno[2,1-c] pyrazole-1-yl)-1-méthyléthylamine.

**8.** Fumarate (1:1) de la (RS)-2-(7-méthoxy-4,4-diméthyl-1,4-dihydroindéno-[2,1-c]pyrazole-1-yl)-1-méthyléthylamine.

**9.** Fumarate (1:1) de la (RS)-2-(7-éthoxy-1,4-dihydroindéno[2,1-c]pyrazole-1-yl)-1-méthyléthylamine.

**10.** Fumarate (1:1) de la (R)-2-(7-méthoxy-1,4-dihydroindéno[2,1-c] pyrazole-1-yl)-1-méthyléthylamine.

**11.** Fumarate (1:0,5) de (RS)-2-(8-méthoxy-1H-benzo[g]indazole-1-yl)-1-méthyléthylamine.

**12.** Composés de formule générale :

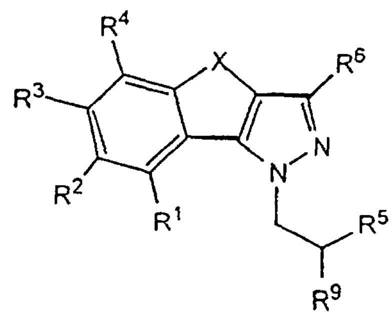

II

dans laquelle $R^1$ à $R^6$ ont les significations données dans la revendication 1, et $R^9$ est un groupe azido, un groupe hydroxy, un groupe acétylamino ou un groupe trifluoracétylamino.

**13.** Médicament contenant un composé selon l'une des revendications 1 à 11 et un matériau excipient inerte d'un point de vue thérapeutique, en particulier pour le traitement ou la prévention des troubles du système nerveux central, des dépressions, des troubles bipolaires, des états d'angoisse, des troubles du sommeil et de la sexualité, des psychoses, de la schizophrénie, des migraines et d'autres états pathologiques liés à une céphalée ou à des douleurs d'une autre nature, des troubles de la personnalité ou des névroses obsessionnelles, des phobies sociales ou des crises paniques, des troubles organiques mentaux, des troubles mentaux de l'enfance, de l'agressivité, des troubles de la mémoire et du comportement lié à l'âge, des manies, de l'obésité, de la boulimie, etc., des lésions du système nerveux sous l'effet de traumatismes, d'accidents vasculaires cérébraux, des maladies neurodégénératives, etc., des troubles cardiovasculaires tels que l'hypertension artérielle, la thrombose, les accidents

vasculaires cérébraux, etc., et des troubles gastrointestinaux, tels qu'un disfonctionnement du transit gastrointestinal.

**14.** Procédé de préparation de composés selon l'une des revendications 1 à 11, **caractérisé en ce que** :

a) on convertit un composé de formule générale :

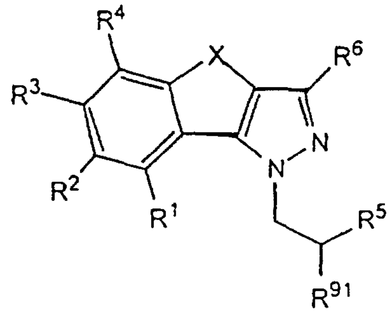

IIa

dans laquelle $R^1$ à $R^6$ ont les significations données dans la revendication 1, et $R^{91}$ est un groupe azido, un groupe acétylamino ou un groupe trifluoracétylamino, en un composé aminé correspondant de formule I, et

b) si on le souhaite, on convertit en un sel acceptable d'un point de vue pharmaceutique le composé obtenu de formule I.

**15.** Composés selon l'une des revendications 1 à 11 pour utilisation en tant que principes actifs thérapeutiques, en particulier pour le traitement ou la prévention des troubles du système nerveux central, des dépressions, des troubles bipolaires, des états d'angoisse, des troubles du sommeil et de la sexualité, des psychoses, de la schizophrénie, des migraines et d'autres états pathologiques liés à une céphalée ou à des douleurs d'une autre nature, des troubles de la personnalité ou des névroses obsessionnelles, des phobies sociales ou des crises paniques, des troubles organiques mentaux, des troubles mentaux de l'enfance, de l'agressivité, des troubles de la mémoire et du comportement liés à l'âge, des manies, de l'obésité, de la boulimie, etc., des lésions du système nerveux sous l'effet de traumatismes, d'accidents vasculaires cérébraux, des maladies neurodégénératives, etc., des troubles cardiovasculaires tels que l'hypertension artérielle, la thrombose, les accidents vasculaires cérébraux, etc., et des troubles gastrointestinaux, tels qu'un disfonctionnement du transit gastrointestinal.